(19) Europäisches Patentamt
European Patent Office
Office européen des brevets

(11) Veröffentlichungsnummer: **0 645 390 A1**

# (12) EUROPÄISCHE PATENTANMELDUNG

(21) Anmeldenummer: **94111739.2**

(22) Anmeldetag: **26.07.94**

(51) Int. Cl.[6]: **C07D 487/04**, A61K 31/505, //(C07D487/04,239:00,239:00)

(30) Priorität: **02.08.93 DE 4325900**

(43) Veröffentlichungstag der Anmeldung: **29.03.95 Patentblatt 95/13**

(84) Benannte Vertragsstaaten: **AT BE CH DE DK ES FR GB GR IE IT LI LU NL PT SE**

(71) Anmelder: **Dr. Karl Thomae GmbH**

**D-88397 Biberach (DE)**

(72) Erfinder: **Heckel, Armin, Dr. Dipl.-Chem.**
**Geschwister-Scholl-Strasse 71**
**D-88400 Biberach (DE)**
Erfinder: **Bamberger, Uwe, Dr. Tierarzt**
**Grünweiler 90**
**D-88416 Ochsenhausen (DE)**
Erfinder: **Mauz, Annerose, Dr., Dipl.-Biol.**
**Habsbergerstrasse 59**
**D-88515 Langenenslingen-Emerfeld (DE)**

(54) **Trisubstituierte Pyrimido/5,4-d/-pyrimidine zur Modulation der Multidrugresistenz, diese Verbindungen enthaltende Arzneimittel und Verfahren zu ihrer Herstellung.**

(57) Die Erfindung betrifft trisubstituierte Pyrimido[5,4-d]pyrimidine der allgemeinen Formel

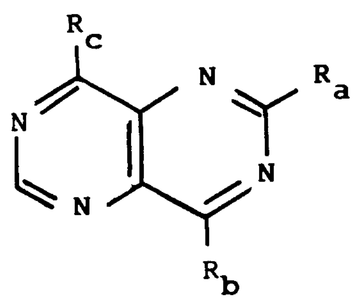

, (I)

in der
$R_a$, $R_b$ und $R_c$ wie im Anspruch 1 definiert sind,
deren Salze, insbesondere für die pharmazeutische Anwendung deren physiologisch verträgliche Salze, welche u.a. wertvolle pharmakologische Eigenschaften aufweisen, insbesondere bei der Chemotherapie eine sensibilisierende Wirkung auf resistente Tumore, deren Verwendung und Verfahren zu ihrer Herstellung.

EP 0 645 390 A1

Die Chemotherapie von malignen Erkrankungen hat nur bei wenigen Erkrankungen Erfolge im Sinne einer Heilung erbracht. Unter anderem verhindern vor Beginn der Behandlung vorhandene oder unter Therapie entstandene Resistenzen bessere Therapieerfolge. Die Ursache und das Erscheinungsbild dieser Resistenzen sind vielfältig. Doch ist häufig eine nicht auf bestimmte chemische oder pharmakologische Gruppen von Wirkstoffen beschränkte pleiotrope Resistenz zu beobachten, die meist auf dem Transport von Wirkstoffen aus Tumorzellen und damit auf einer verminderten intrazellulären Wirksktoffakkummulation beruhen. Ein klinisch bedeutsamer und bis heute der am besten untersuchte Mechanismus der pleiotropen Resistenz beruht auf der Expression des Transportproteins gp170 (P-Glykoprotein) in der Membran von Tumorzellen (siehe Ferguson und Cheng, Critical Issues Relating to Clinical Drug Resistance, Cancer Bulletin 41: 7-13, 1989). Dieses Transportprotein besitzt eine Spezifität für lipophile Substanzen und beeinflußt somit die intrazelluäre Konzentration von heute klinisch bedeutsamen Zytostatika der Klasse der Vinkaalkaloide, der Anthrazyklin-Antibiotika, der Epipodophylotoxine und anderer Naturstoffe (siehe van der Bliek and Borst, Multidrug Resistance, Advances in Cancer Research 52: 165-203, 1989).

Es wurde nun gefunden, daß trisubstituierte Pyrimido[5,4-d]pyrimidine der allgemeinen Formel

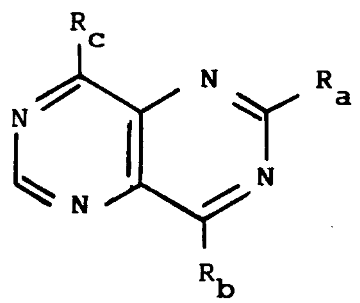

, (I)

wertvolle Eigenschaften aufweisen. Diese stellen, wenn $R_a$ eine >CO- oder >($R_4$O-C-O$R_5$)-Gruppe darstellt, wertvolle Zwischenprodukte zur Herstellung der übrigen Verbindungen der allgemeinen Formel I dar, welche wertvolle pharmakologische Eigenschaften aufweisen, insbesondere bei der Chemotherapie eine sensibilisierende Wirkung auf resistente Tumore.

Gegenstand der vorliegenden Erfindung sind somit die neuen Verbindungen der obigen allgemeinen Formel I, deren Enantiomere, deren Salze, insbesondere für die pharmazeutische Anwendung deren physiologisch verträgliche Salze, diese Verbindungen enthaltende Arzneimittel, deren Verwendung und Verfahren zu ihrer Herstellung.

In der obigen allgemeinen Formel bedeutet

$R_a$ eine Pyrrolidino-, Piperidino- oder Hexamethyleniminogruppe, wobei eine Methylengruppe in einer Pyrrolidinogruppe in 3-Stellung oder in einer Piperidino- und Hexamethyleniminogruppe in 3- oder 4-Stellung jeweils durch eine >C$R_1$-A-($R_2$N$R_3$)-, >CO- oder >($R_4$O-C-O$R_5$)-Gruppe oder durch eine Gruppe der Formel

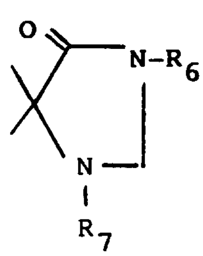

ersetzt ist, in denen

A eine Kohlenstoff-Stickstoff-Bindung oder eine Alkylengruppe mit 1 bis 3 Kohlenstoffatomen,

$R_1$ ein Wasserstoffatom oder eine Alkylgruppe mit 1 bis 3 Kohlenstoffatomen,

$R_2$ ein Wasserstoffatom oder eine gegebenenfalls durch eine Phenylgruppe substituierte Alkylgruppe mit 1 bis 3 Kohlenstoffatomen,

$R_3$ ein Wasserstoffatom oder eine gegebenenfalls durch eine Phenylgruppe substituierte Alkylgruppe mit 1 bis 4 Kohlenstoffatomen, in der die Phenylgruppe durch ein Fluor-, Chlor- oder Bromatom, durch eine Alkyl- oder Alkoxygruppe mit jeweils 1 bis 3 Kohlenstoffatomen mono- oder disubstituiert sein kann, wobei die Substituenten gleich oder verschieden sein können,

eine durch eine Carboxy-, Alkoxycarbonyl-, Cyano-, Aminocarbonyl-, Alkylaminocarbonyl- oder Dialkylaminocarbonylgruppe oder durch zwei Phenylgruppen substituierte Alkylgruppe mit 1 bis 4 Kohlenstoffatomen, wobei die vorstehend erwähnten Alkyl- und Alkoxyteile jeweils 1 bis 3 Kohlenstoffatome enthalten können,
eine Alkylgruppe mit 2 bis 4 Kohlenstoffatomen, die in 2-, 3- oder 4-Stellung durch eine Hydroxy-, Amino-, Alkylamino- oder Dialkylaminogruppe substituiert sein kann, wobei die vorstehend erwähnten Alkylteile jeweils 1 bis 3 Kohlenstoffatome enthalten können,
eine Alkoxycarbonylgruppe mit insgesamt 2 bis 4 Kohlenstoffatomen oder
$R_2$ und $R_3$ zusammen mit dem dazwischenliegenden Stickstoffatom eine Cycloalkyleniminogruppe mit 3 bis 7 Ring gliedern, eine Morpholino-, Thiomorpholino-, 1-Oxido-thiomorpholino- oder 1,1-Dioxido-thiomorpholinogruppe,
$R_4$ und $R_5$, die gleich oder verschieden sein können, Alkylgruppen mit jeweils 1 bis 3 Kohlenstoffatomen oder
$R_4$ und $R_5$ zusammen eine n-Alkylengruppe mit 2 oder 3 Kohlenstoffatomen,
$R_6$ ein Wasserstoffatom oder eine Alkylgruppe mit 1 bis 3 Kohlenstoffatomen und
$R_7$ ein Wasserstoffatom, eine Alkylgruppe mit 1 bis 3 Kohlenstoffatomen oder eine Phenylgruppe darstellen,
$R_b$ und $R_c$, die gleich oder verschieden sein können, jeweils eine cyclische Alkylenimino- oder Alkenyleniminogruppe mit 5 bis 7 Ringgliedern, an die über die 2-, 3- oder 3-, 4-Stellungen eine 1,4-Butadienylbrücke angefügt sein kann, welche durch eine Methylendioxygruppe monosubstituiert oder durch Fluor-, Chlor- oder Bromatome, durch Alkyl-, Alkoxy-, Amino-, Alkylamino-, Dialkylamino- oder Alkanoylaminogruppen mono-, di- oder trisubstituiert sein können, wobei die Alkyl- und Alkoxyteile jeweils 1 bis 3 Kohlenstoffatome und der Alkanoylteil 2 oder 3 Kohlenstoffatome enthalten können sowie die Substituenten gleich oder verschieden sein können, eine Morpholino-, Thiomorpholino-, 1-Oxido-thiomorpholino-, 1,1-Dioxido-thiomorpholino- oder ($R_8NR_9$)-Gruppe, in der
$R_8$ ein Wasserstoffatom oder eine Alkylgruppe mit 1 bis 4 Kohlenstoffatomen, welche durch eine Phenyl-, Cyano-, Carboxy- oder Alkoxycarbonylgruppe oder in 2-, 3- oder 4-Stellung durch eine Amino-, Alkylamino-, Dialkylamino-, Alkanoylamino-, Benzoylamino- oder Phenylsulfonylaminogruppe substituiert sein kann, wobei die vorstehend erwähnten Alkyl-, Alkoxy- und Alkanoylteile jeweils 1 bis 3 Kohlenstoffatome enthalten können und der Phenylkern jeweils durch ein Fluor-, Chlor- oder Bromatom, durch eine Alkyl- oder Alkoxygruppe mit jeweils 1 bis 3 Kohlenstoffatome mono- oder disubstituiert sein und die Substituenten gleich oder verschieden sein können, und
$R_9$ ein Wasserstoffatom, eine gegebenenfalls durch eine Phenyl- oder Naphthylgruppe substituierte Alkylgruppe mit 1 bis 4 Kohlenstoffatomen, in der die Phenyl- und Naphthylgruppe jeweils durch ein Fluor-, Chlor- oder Bromatom, durch eine Alkyl-, Alkoxy-, Amino-, Alkylamino- oder Dialkylaminogruppe mono- oder disubstituiert sein kann, wobei die Substituenten gleich oder verschieden sein können und jeder Alkyl- und Alkoxyteil jeweils 1 bis 3 Kohlenstoffatomen enthalten kann, eine Alkanoylaminogruppe mit 1 bis 5 Kohlenstoffatomen oder eine Anthracenylgruppe darstellen,
oder $R_c$ auch eine gegebenenfalls durch eine Phenylgruppe substituierte Alkoxy- oder Alkylthiogruppe mit jeweils 1 bis 3 Kohlenstoffatomen.

Für die bei der Definition der Reste eingangs erwähnten Bedeutungen kommt beispielsweise für
$R_a$ die der 3-Amino-piperidino-, 4-Methylamino-piperidino-, 4-Dimethylamino-piperidino-, 4-Ethylamino-piperidino-, 4-Diethylamino-piperidino-, 4-(N-Methyl-ethylamino)-piperidino-, 4-n-Dipropylamino-piperidino-, 4-Benzylamino-piperidino-, 4-(N-Methyl-benzylamino)-piperidino-, 4-(N-Ethyl-benzylamino)-piperidino-, 4-(N-Isopropyl-benzylamino)-piperidino-, 4-Pyrrolidino-piperidino-, 4-Piperidino-piperidino-, 4-(2,2-Diphenyl-ethyl)-piperidino-, 4-(3,3-Diphenyl-propyl)-piperidino-, 4-Carboxymethylamino-piperidino-, 4-Methoxycarbonylamino-piperidino-, 4-Ethoxycarbonylamino-piperidino-, 4-n-Propoxycarbonylamino-piperidino-,4-Dimethylaminocarbonylmethylamino-piperidino-, 4-(2-Dimethylaminocarbonylethyl-amino)-piperidino-, 4-(3-Dimethylaminocarbonylpropyl-amino)-piperidino-, 4-Diethylaminocarbonylmethylamino-piperidino-, 4-(2-Diethylaminocarbonylethyl-amino)-piperidino-, 4-(3-Diethylaminocarbonylpropyl-amino)-piperidino-, 4-Di-n-propylaminocarbonylmethylamino-piperidino-, 4-(2-Di-n-propylaminocarbonylethyl-amino)-piperidino-, 4-(3-Di-n-propylaminocarbonylpropyl-amino)-piperidino-, 4-(N-Carboxymethyl-methylamino)-piperidino-, 4-(N-Methoxycarbonylmethylamino)-piperidino-, 4-(N-Ethoxycarbonyl-methylamino)-piperidino-, 4-(N-n-Propoxycarbonyl-methylamino)-piperidino-, 4-(N-Dimethylaminocarbonylmethyl-methylamino)-piperidino-, 4-[N-(2-Dimethylaminocarbonylethyl)-methylamino]-piperidino-, 4-[N-(3-Dimethylaminocarbonylpropyl)-methylamino]-piperidino-, 4-(N-Diethylaminocarbonylmethyl-methylamino)-piperidino-, 4-[N-(2-Diethylaminocarbonylethyl)-methylamino]-piperidino-, 4-[N-(3-Diethylaminocarbonylpropyl)-methylamino]iperidino-, 4-(N-Di-n-propylaminocarbonylmethyl-methylamino)-piperidino-, 4-[N-(2-Di-n-propylaminocarbonylethyl)-methylamino]-piperidino-, 4-[N-(3-Di-n-propylaminocarbonylpropyl)-methylamino]-piperidino-, 4-(N-Carboxymethyl-ethylamino)-piperidino-, 4-(N-Methoxycarbonyl-ethylamino)-piperidino-, 4-(N-Ethoxycarbonyl-ethylamino)-piperidino-, 4-(N-n-Propoxycarbonyl-ethylamino)-piperidino-, 4-(N-Dimethylaminocarbonylmethyl-ethylamino)-piperidino-, 4-[N-(2-Dimethylaminocarbonylethyl)-ethylamino]-piperidino-, 4-[N-(3-Dimethylaminocarbonylpropyl)-ethylamino]-piperidino-, 4-(N-Diethylaminocarbonylmethyl-ethylamino)-piperidino-, 4-[N-(2-Diethylaminocarbonylethyl)-ethylamino]-piperidino-, 4-[N-(3-Diethylaminocarbonylpropyl)-ethylamino]-piperidino-, 4-(N-Di-n-propylaminocarbonylmethyl-ethylamino)-piperidino-, 4-[N-(2-Di-n-propylaminocarbonylethyl)-ethylamino]-piperidino-, 4-[N-(3-Di-n-propylaminocarbonylpropyl)-ethylamino]-piperidino-, 4-(N-Carboxymethyl-isopropylamino)-piperidino-, 4-(N-Methoxycarbonyl-n-propylmethylamino)-piperidino-, 4-(N-Ethoxycarbonyl-n-propylamino)-piperidino-, 4-(N-n-Propoxycarbonyl-isopropylamino)-piperidino-, 4-(N-Dimethylaminocarbonylmethyl-n-propylamino)-piperidino-, 4-[N-(2-Dimethylaminocarbonylethyl)-n-propylamino]-piperidino-, 4-[N-(3-Dimethylaminocarbonylpropyl)-n-propylamino]-piperidino-, 4-(N-Diethylaminocarbonylmethyl-n-propylamino)-piperidino-, 4-[N-(2-Diethylaminocarbonylethyl)-n-propylamino]-piperidino-, 4-[N-(3-Diethylaminocarbonylpropyl)-isopropylamino]-piperidino-, 4-(N-Di-n-propylaminocarbonylmethyl-isopropylamino)-piperidino-, 4-[N-(2-Di-n-propylaminocarbonylethyl)-n-propylamino]-piperidino-, 4-[N-(3-Di-n-propylaminocarbonylpropyl)-n-propylamino]-piperidino-,4-[N-(2-Aminoethyl)-amino]-piperidino-, 4-[N-(2-Methylamino-ethyl)-amino]-piperidino-, 4-[N-(2-Ethylaminoethyl)-amino]-piperidino-, 4-[N-(2-Isopropylamino-ethyl)-amino]-piperidino-, 4-[N-(2-Dimethylamino-ethyl)-amino]-piperidino-, 4-[N-(2-Diethylamino-ethyl)-amino]-piperidino-, 4-[N-(2-Di-n-propylamino-ethyl)-amino]-piperidino-, 4-[N-(3-Amino-propyl)-amino]-piperidino-, 4-[N-(3-Methylamino-propyl)-amino]-piperidino-, 4-[N-(3-Ethylamino-propyl)-amino]-piperidino-, 4-[N-(3-Isopropylamino-propyl)-amino]-piperidino-, 4-[N-(3-Dimethylamino-propyl)-amino]-piperidino-, 4-[N-(3-Diethylamino-propyl)-amino]-piperidino-, 4-[N-(3-Di-n-propylamino-propyl)-amino]-piperidino-, 4-[N-(2-Aminoethyl)-methylamino]-piperidino-, 4-[N-(2-Methylamino-ethyl)-methylamino]-piperidino-, 4-[N-(2-Ethylamino-ethyl)-methylamino]-piperidino-, 4-[N-(2-Isopropylamino-ethyl)-methylamino]-piperidino-, 4-[N-(2-Dimethylamino-ethyl)-methylamino]-piperidino-, 4-[N-(2-Diethylamino-ethyl)-methylamino]-piperidino-, 4-[N-(2-Di-n-propylamino-ethyl)-methylamino]-piperidino-, 4-[N-(3-Amino-propyl)-methylamino]-piperidino-, 4-[N-(3-Methylamino-propyl)-methylamino]-piperidino-, 4-[N-(3-Ethylamino-propyl)-methylamino]-piperidino-, 4-[N-(3-Isopropylamino-propyl)-methylamino]-piperidino-, 4-[N-(3-Dimethylamino-propyl)-methylamino]-piperidino-, 4-[N-(3-Diethylaminopropyl)-methylamino]-piperidino-, 4-[N-(3-Di-n-propylaminopropyl)-methylamino]-piperidino-, 4-[N-(2-Amino-ethyl)-ethylamino]-piperidino-, 4-[N-(2-Methylamino-ethyl)-ethylamino]-piperidino-, 4-[N-(2-Ethylamino-ethyl)-ethylamino]-piperidino-, 4-[N-(2-Isopropylamino-ethyl)-ethylamino]-piperidino-, 4-[N-(2-Dimethylamino-ethyl)-ethylamino]-piperidino-, 4-[N-(2-Diethylamino-ethyl)-ethylamino]-piperidino-, 4-[N-(2-Di-n-propylamino-ethyl)-ethylamino]-piperidino-, 4-[N-(3-Aminopropyl)-ethylamino]-piperidino-, 4-[N-(3-Methylamino-propyl)-ethylamino]-piperidino-, 4-[N-(3-Ethylamino-propyl)-ethylamino]-piperidino-, 4-[N-(3-Isopropylamino-propyl)-ethylamino]-piperidino-, 4-[N-(3-Dimethylamino-propyl)-ethylamino]-piperidino-, 4-[N-(3-Diethylamino-propyl)-ethylamino]-piperidino-, 4-[N-(3-Di-n-propylamino-propyl)-ethylamino]-piperidino-, 4-[N-(2-Amino-ethyl)-n-propylamino]-piperidino-, 4-[N-(2-Methylamino-ethyl)-isopropylamino]-piperidino-, 4-[N-(2-Ethylamino-ethyl)-isopropylamino]-piperidino-, 4-[N-(2-Isopropylamino-ethyl)-n-propylamino]-piperidino-, 4-[N-(2-Dimethylamino-ethyl)-n-propylamino]-piperidino-, 4-[N-(2-Diethylamino-ethyl)-n-propylamino]-piperidino-, 4-[N-(2-Di-n-propylamino-ethyl)-n-propylamino]-piperidino-, 4-[N-(3-Amino-propyl)-n-propylamino]-piperidino-, 4-[N-(3-Methylamino-propyl)-n-propylamino]-piperidino-, 4-[N-(3-Ethylamino-propyl)-n-propylamino]-piperidino-, 4-[N-(3-Isopropylamino-propyl)-n-propylamino]-piperidino-, 4-[N-(3-Dimethylamino-propyl)-n-propylamino]-piperidino-, 4-[N-(3-Diethylamino-propyl)-n-propylamino]-piperidino-, 4-[N-(3-Di-n-propylamino-propyl)-n-propylamino]-piperidino-, 3-Amino-pyrrolidino-, 3-Methylamino-pyrrolidino-, 3-Dimethylamino-pyrrolidino-, 3-Diethylamino-pyrrolidino-, 3-Amino-hexamethylenimino-, 4-Amino-hexamethylenimino-, 3-Methylamino-hexamethylenimino-, 4-Methylamino-hexamethylenimino-, 3-Dimethylamino-hexamethylenimino-, 4-Dimethylamino-hexamethylenimino-, 3-Diethylamino-hexamethylenimino- oder 4-Diethylamino-hexamethylenimino-Gruppe,

für $R_b$ und $R_c$ jeweils die der Pyrrolidino-, Piperidino-, Hexamethylenimino-, Morpholino-, Thiomorpholino-, 1-Oxido-thiomorpholino-, 1,1-Dioxido-thiomorpholino-, 1,2,3,4-Tetrahydro-isochinolino-, 1,2,3,4-Tetrahydro-6,7-dimethoxy-isochinolino-, Methylamino-, Ethylamino-, n-Propylamino-, Isopropylamino-, Dimethylamino-, Diethylamino-, Di-n-propylamino-, Di-isopropylamino-, Benzylamino-, 4-Fluor-benzylamino-, 4-Chlor-benzylamino-, 4-Brom-benzylamino-, 4-Methyl-benzylamino-, 4-Methoxy-benzylamino-, Napht-1-yl-methylamino-, Napht-2-yl-methylamino-, 4-Fluor-napht-1-yl-methylamino-, 4-Chlor-napht-1-yl-methylamino-, 4-Brom-napht-2-yl-methylamino-, 4-Methyl-napht-2-yl-methylamino-, 4-Methoxy-naphtl-yl-methylamino-, 4-Dimethylamino-napht-2-yl-methylamino-, N-Benzyl-methylamino-, N-(4-Fluor-benzyl)-methylamino-, N-(4-Chlor-benzyl)-methylamino-,N-(4-Brom-benzyl)-methylamino-, N-(4-Methyl-benzyl)-methylamino-,N-(4-Methoxy-benzyl)-methylamino-, N-(Napht-1-yl-methyl)-methylamino-, N-(Napht-2-yl-methyl)-methylamino-, N-(4-Fluor-napht-1-yl-methyl)-methylamino-, N-(4-Chlor-napht-1-yl-methyl)-methylamino-, N-(4-Brom-napht-2-yl-methyl)-methylamino-, N-(4-Methyl-napht-2-yl-methyl)-methylamino-, N-(4-Methoxy-napht-1-yl-methyl)-methylamino-, N-(4-Dimethylamino)-napht-2-yl-methyl)-methylamino-, N-Benzyl-ethylamino-, N-(4-Fluor-benzyl)-ethylamino-, N-(4-Chlor-benzyl)-ethylamino-, N-(4-Brom-benzyl)-ethylamino-, N-(4-Methyl-benzyl)-ethylamino-, N-(4-Methoxy-benzyl)-ethylamino-, N-(Napht-1-yl-methyl)-ethylamino-, N-(Napht-2-yl-methyl)-ethylamino-, N-(4-Fluor-napht-1-yl-methyl)-ethylamino-, N-(4-Chlor-napht-1-yl-methyl)-ethylamino-, N-(4-Brom-napht-2-yl-methyl)-ethylamino-, N-(4-Methyl-napht-2-yl-methyl)-ethylamino-, N-(4-Methoxy-napht-1-yl-methyl)-ethylamino-, N-(4-Dimethylamino)-napht-2-yl-ethyl)-methylamino-, N-Benzyl-n-propylamino-, N-(4-Fluor-benzyl)-n-propylamino-, N-(4-Chlor-benzyl)-n-propylamino-, N-(4-Brom-benzyl)-n-propylamino-, N-(4-Methyl-benzyl)-n-propylamino-, N-(4-Methoxy-benzyl)-n-propylamino-, N-(Napht-1-yl-methyl)-n-propylamino-, N-(Napht-2-yl-methyl)-n-propylamino-, N-(4-Fluor-napht-1-yl-methyl)-n-propylamino-, N-(4-Chlor-napht-1-yl-methyl)-n-propylamino-, N-(4-Brom-napht-2-yl-methyl)-n-propylamino-, N-(4-Methyl-napht-2-yl-methyl)-n-propylamino-, N-(4-Methoxy-napht-1-yl-methyl)-n-propylamino-, N-(4-Dimethylamino)-napht-2-yl-ethyl)-n-propylamino-, N-Benzyl-isopropylamino-, N-(4-Fluor-benzyl)-isopropylamino-, N-(4-Chlor-benzyl)-isopropylamino-, N-(4-Brom-benzyl)-isopropylamino-, N-(4-Methyl-benzyl)-isopropylamino-, N-(4-Methoxy-benzyl)-isopropylamino-, N-(Napht-1-yl-methyl)-isopropylamino-, N-(Napht-2-yl-methyl)-isopropylamino-, N-(4-Fluor-napht-1-yl-methyl)-isopropylamino-, N-(4-Chlor-napht-1-yl-methyl)-isopropylamino-, N-(4-Brom-napht-2-yl-methyl)-isopropylamino-, N-(4-Methyl-napht-2-yl-methyl)-isopropylamino-, N-(4-Methoxy-napht-1-yl-methyl)-isopropylamino-, N-(4-Dimethylamino)-napht-2-yl-ethyl)-isopropylamino-, N-(2-Phenyl-ethyl)-amino-, N-(3-Phenyl-propyl)-amino-, N-(2-Phenyl-ethyl)-methylamino-, N-(3-Phenyl-propyl)-methylamino-, N-(2-Phenyl-ethyl)-ethylamino-, N-(3-Phenyl-propyl)-ethylamino-, N-(2-Phenyl-ethyl)-n-propylamino-, N-(3-Phenyl-propyl)-n-propylamino-, N-(2-Phenyl-ethyl)-isopropylamino-, N-(3-Phenyl-propyl)-isopropylamino-, N-(2-Cyano-ethyl)-amino-, N-(3-Cyano-propyl)-amino-, N-(2-Amino-ethyl)-amino-, N-(2-Methylamino-ethyl)-amino-, N-(2-Dimethylamino-ethyl)-amino-, N-(2-Diethylamino-ethyl)-amino-, N-(2-Di-n-propylamino-ethyl)-amino-, N-(2-Diisopropylamino-ethyl)-amino-, N-(2-Acetylamino-ethyl)-amino-,N-(2-Propionylamino-ethyl)-amino-, N-(2-Benzoylamino-ethyl)-amino-,N-(2-Benzolsulfonylamino-ethyl)-amino-, N-(2-Toluolsulfonylamino-ethyl)-amino-, N-(2-Cyano-ethyl)-methylamino-, N-(3-Cyano-propyl)-methylamino-, N-(2-Amino-ethyl)-methylamino-, N-(2-Methylamino-ethyl)-methylamino-, N-(2-Dimethylamino-ethyl)-methylamino-, N-(2-Diethylamino-ethyl)-methylamino-, N-(2-Di-n-propylamino-ethyl)-methylamino-, N-(2-Diisopropylamino-ethyl)-methylamino-, N-(2-Acetylamino-ethyl)-methylamino-, N-(2-Propionylamino-ethyl)-methylamino-, N-(2-Benzoylamino-ethyl)-methylamino-, N-(2-Benzolsulfonylamino-ethyl)-methylamino-, N-(2-Toluolsulfonylamino-ethyl)-methylamino-, N-(2-Cyano-ethyl)-ethylamino-, N-(3-Cyano-propyl)-ethylamino-, N-(2-Amino-ethyl)-ethylamino-, N-(2-Methylamino-ethyl)-ethylamino-,N-(2-Dimethylamino-ethyl)-ethylamino-, N-(2-Diethylamino-ethyl)-ethylamino-, N-(2-Di-n-propylamino-ethyl)-ethylamino- N-(2-Diisopropylamino-ethyl)-ethylamino-, N-(2-Acetylamino-ethyl)-ethylamino-, N-(2-Propionylamino-ethyl)-ethylamino-, N-(2-Benzoylamino-ethyl)-ethylamino-, N-(2-Benzolsulfonylamino-ethyl)-ethylamino-, N-(2-Toluolsulfonylamino-ethyl)-ethylamino-, N-(2-Cyano-ethyl)-n-propylamino-, N-(3-Cyano-propyl)-isopropylamino-, N-(2-Aminoethyl)-n-propylamino-,N-(2-Methylamino-ethyl)-n-propylamino-, N-(2-Dimethylamino-ethyl)-isopropylamino-, N-(2-Diethylamino-ethyl)-n-propylamino-, N-(2-Di-n-propylamino-ethyl)-n-propylamino-, N-(2-Diisopropylamino-ethyl)-n-propylamino-, N-(2-Acetylamino-ethyl)-n-propylamino-, N-(2-Propionylamino-ethyl)-n-propylamino-, N-(2-Benzoylamino-ethyl)-n-propylamino-, N-(2-Benzolsulfonylamino-ethyl)-n-propylamino-, N-(2-Toluolsulfonylamino-ethyl)-isopropylamino-, N-(2-Cyano-ethyl)-benzylamino-, N-(3-Cyano-propyl)-benzylamino-, N-(2-Amino-ethyl)-benzylamino-, N-(2-Methylamino-ethyl)-benzylamino-, N-(2-Dimethylamino-ethyl)-benzylamino, N-(2-Diethylamino-ethyl)-benzylamino-, N-(2-Di-n-propylamino-ethyl)-benzylamino-, N-(2-Diisopropylamino-ethyl)-benzylamino, N-(2-Acetylamino-ethyl)-benzylamino-, N-(2-Propionylamino-ethyl)-benzylamino-, N-(2-Benzoylamino-ethyl)-benzylamino, N-(2-Benzolsulfonylamino-ethyl)-benzylamino-, N-(2-Toluolsulfonylamino-ethyl)-benzylamino-, N-Carboxymethyl-amino-, N-Methoxycarbonylmethyl-amino-, N-Ethoxycarbonylmethylamino-, N-Isopropoxycarbonylmethyl-amino-, N-(2-Carboxy-ethyl)-amino-, N-(2-Methoxycarbonyl-ethyl)-amino-, N-(2-Ethoxycarbonyl-ethyl)-amino-, N-(2-n-Propoxycarbonyl-ethyl)-amino-, N-(3-Carboxy-propyl)-amino-,N-(3-Methoxycarbonyl-propyl)-amino-, N-(3-Ethoxycarbonyl-propyl)-amino-, N-(3-Isopropoxycarbonyl-propyl)-amino-, N-Carboxymethyl-methylamino-, N-Methoxycarbonyl-methyl-methylamino-, N-Ethoxycarbonylethyl-methylamino-, N-Isopropoxycarbonylmethyl-methylamino-,N-(2-Carboxy-ethyl)-methylamino-, N-(2-Methoxycarbonyl-ethyl)-methylamino-, N-(2-Ethoxycarbonyl-ethyl)-methylamino-, N-(2-n-Propoxycarbonyl-ethyl)-methylamino-, N-(3-Carboxy-propyl)-methylamino-, N-(3-Methoxycarbonyl-propyl)-methylamino-, N-(3-Ethoxycarbonyl-propyl)-methylamino-, N-(3-Isopropoxycarbonyl-propyl)-methylamino-, N-Carboxymethyl-ethylamino-, N-Methoxycarbonylmethyl-ethylamino-, N-Ethoxycarbonylmethyl-ethylamino-, N-Isopropoxycarbonylmethyl-ethylamino-,N-(2-Carboxy-ethyl)-ethylamino-, N-(2-Methoxycarbonyl-ethyl)-ethylamino-, N-(2-Ethoxycarbonyl-ethyl)-ethylamino-, N-(2-n-Propoxycarbonyl-ethyl)-ethylamino-, N-(3-Carboxy-propyl)-ethylamino-, N-(3-Methoxycarbonyl-propyl)-ethylamino-, N-(3-Ethoxycarbonyl-propyl)-ethylamino-, N-(3-Isopropoxycarbonyl-propyl)-ethylamino-, N-Carboxymethyl-n-propylamino-, N-Methoxycarbonylmethyl-n-propylamino-, N-Ethoxycarbonylmethyl-n-propylamino-, N-Isopropoxycarbonylmethyl-isopropylamino-, N-(2-Carboxy-ethyl)-n-propylamino-, N-(2-Methoxycarbonyl-ethyl)-isopropylamino, N-(2-Ethoxycarbonyl-ethyl)-n-propylamino-, N-(2-n-Propoxycarbonyl-ethyl)-n-propylamino-, N-(3-Carboxypropyl)-n-propylamino-, N-(3-Methoxycarbonyl-propyl)-isopropylamino-, N-(3-Ethoxycarbonyl-propyl)-n-propylamino-, N-(3-Isopropoxycarbonyl-propyl)-n-propylamino-, N-Carboxymethyl-benzylamino-, N-Methoxycarbonylmethyl-benzylamino-, N-Ethoxycarbonylmethyl-benzylamino-, N-Isopropoxycarbonylmethyl-benzylamino, N-(2-Carboxy-ethyl)-benzylamino-,N-(2-Methoxycarbonyl-ethyl)-benzylamino-, N-(2-Ethoxycarbonyl-ethyl)-benzylamino-, N-(2-n-Propoxycarbonyl-ethyl)-benzylamino-, N-(3-Carboxy-propyl)-benzylamino-, N-(3-Methoxycarbonyl-propyl)-benzylamino-, N-(3-Ethoxycarbonyl-propyl)-benzylamino-, N-(3-Isopropoxycarbonyl-propyl)-benzylamino-, N-Anthracen-9-yl-amino-, N-Anthracen-9-yl-methylamino-, N-Anthracen-9-yl-ethylamino-, N-Anthracen-9-yl-n-propylamino-, N-Anthracen-9-yl-isopropylamino-, N-Anthracen-9-yl-benzylamino-, 5-Methoxy-1,2,3,4-tetrahydro-isochinolin-2-yl-, 6-Methoxy-1,2,3,4-tetrahydro-isochinolin-2-yl-, 7-Methoxy-1,2,3,4-tetrahydro-isochinolin-2-yl-, 6,7-Methylendioxy-1,2,3,4-tetrahydro-isochinolin-2-yl-, 5-Acetamido-1,2,3,4-tetrahydro-isochinolin-2-yl- oder 5-Propionylamino-1,2,3,4-tetrahydro-isochinolin-2-yl-Gruppe und

für $R_c$ zusätzlich die der Methoxy-, Ethoxy-, n-Propoxy-, Isopropoxy-, Benzyloxy-, 2-Phenylethoxy-, 3-Phenylethoxy-, Methylthio-, Ethylthio-, n-Propylthio-, Isopropylthio-, Benzylylthio-, 2-Phenylethylthio- oder 3-Phenylethylthio-Gruppe in Betracht.

Bevorzugte Verbindungen der obigen allgemeinen Formel I sind diejenigen, in denen

$R_a$ eine Pyrrolidino- oder Piperidinogruppe, in der eine Methylengruppe in 4-Stellung durch eine $>CR_1$-A-($R_2NR_3$)-, >CO- oder >($R_4$O-C-O$R_5$)-Gruppe oder durch eine Gruppe der Formel

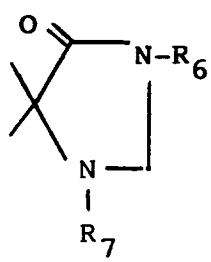

ersetzt ist, in denen

A eine Kohlenstoff-Stickstoff-Bindung oder eine Methylengruppe,

$R_1$ ein Wasserstoffatom,

$R_2$ ein Wasserstoffatom, eine Methyl- oder Ethylgruppe,

$R_3$ ein Wasserstoffatom,

eine gegebenenfalls durch eine Phenyl-, Carboxy-, Methoxycarbonyl-, Dimethylamino- oder Dimethylaminocarbonylgruppe oder durch zwei Phenylgruppen substituierte Alkylgruppe mit 1 bis 3 Kohlenstoffatomen,

eine Alkylgruppe mit 2 oder 3 Kohlenstoffatomen, die in 2- oder 3-Stellung durch eine Dimethylaminogruppe substituiert ist, eine Alkoxycarbonylgruppe mit insgesamt 2 bis 4 Kohlenstoffatomen oder

$R_2$ und $R_3$ zusammen mit dem dazwischenliegenden Stickstoffatom eine Pyrrolidino- oder Piperidinogruppe,

$R_4$ und $R_5$ zusammen eine Ethylengruppe,

$R_6$ ein Wasserstoffatom und

$R_7$ eine Phenylgruppe darstellen,

$R_b$ eine Dialkylaminogruppe, in der jeder Alkylteil 1 bis 3 Kohlenstoffatome enthalten kann, eine Methylaminogruppe, die am Stickstoffatom durch eine Benzyl- oder Naphthylgruppe substituiert ist, eine Piperidino-, Morpholino- oder 1,2,3,4-Tetrahydro-isochinolyl-Gruppeund

$R_c$ eine gegebenenfalls am Stickstoffatom durch eine Alkylgruppe mit 1 bis 3 Kohlenstoffatomen substituierte Benzylaminogruppe, die im Phenylkern durch ein Fluor-, Chlor- oder Bromatom, durch eine Methyl- oder Methoxygruppe und im Alkylteil durch eine Phenyl-, Carboxy-, Methoxycarbonyl-, Ethoxycarbonyl- oder Cyanogruppe oder auch in 2- oder 3-Stellung durch eine Amino-, Acetylamino-, Benzoylamino- oder p-Toluolsulfonylaminogruppe substituiert sein kann, eine gegebenenfalls am Stickstoffatom durch eine Methylgruppe substituierte Naphthylaminogruppe, die im Naphthylkern durch eine Methoxy- oder Dimethylaminogruppe substituiert sein kann, eine gegebenenfalls durch ein Fluor-, Chlor- oder Bromatom, durch eine Methyl-, Methoxy-, Methylendioxy- oder Acetamidogruppe substituierte 1,2,3,4-Tetrahydro-isochinolyl-Gruppe, eine Morpholino-, N-Methyl-cyclohexyl-methylamino- oder Benzyloxygruppe bedeuten, und

deren Salze.

Besonders bevorzugte Verbindungen der obigen allgemeinen Formel I sind diejenigen, in denen $R_a$ eine Piperidinogruppe, in der eine Methylengruppe in 4-Stellung durch eine >$CR_1$-A-($R_2NR_3$)-Gruppe ersetzt ist, in der

A eine Kohlenstoff-Stickstoff-Bindung oder eine Methylengruppe,

$R_1$ ein Wasserstoffatom,

$R_2$ ein Wasserstoffatom, eine Methyl- oder Ethylgruppe,

$R_3$ ein Wasserstoffatom,

eine durch zwei Phenylgruppen substituierte Alkylgruppe mit 2 oder 3 Kohlenstoffatomen,

eine Alkylgruppe mit 1 bis 3 Kohlenstoffatomen, die in 2- oder 3-Stellung durch eine Dimethylaminogruppe substituiert sein kann,

eine Methoxycarbonylgruppe oder

$R_2$ und $R_3$ zusammen mit dem dazwischenliegenden Stickstoffatom eine Piperidinogruppe darstellen,

$R_b$ eine Dimethylaminogruppe, in der eine Methylgruppe durch einen Benzyl- oder Naphthylmethylrest substituiert ist, eine Piperidino- oder Morpholinogruppe und

$R_c$ eine Dimethylaminogruppe, in der eine Methylgruppe durch einen gegebenenfalls im Phenylkern durch ein Fluor-, Chlor- oder Bromatom, durch eine Methyl- oder Methoxygruppe substituierten Benzylrest oder durch einen Naphthylmethylrest substituiert ist, eine 1,2,3,4-Tetrahydro-6,7-dimethoxy-isochinolinyl-, 1,2,3,4-Tetrahydro-6,7-methylendioxy-isochinolinyl-, Morpholino- oder N-(3-Benzoylamino-propyl)-benzylaminogruppe, wobei die Reste $R_b$ und $R_c$ verschieden sind, bedeuten, und deren Salze.

Erfindungsgemäß erhält man die neuen Verbindungen der allgemeinen Formel I nach folgenden Verfahren:

a) Umsetzung einer Verbindung der allgemeinen Formel

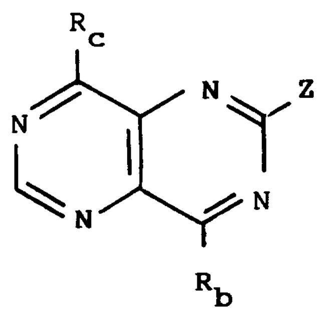

, (II)

in der

$R_b$ und $R_c$ wie eingangs definiert sind und

Z eine Austrittsgruppe wie ein Halogenatom, z.B. ein Chlor- oder Bromatom, eine Phenoxy- oder Methylsulfonylgruppe darstellt, mit einer Verbindung der allgemeinen Formel

$R_a$ - H , (III)

in der

$R_a$ wie eingangs definiert ist.

Die Umsetzung wird zweckmäßigerweise in einem inerten Lösungsmittel wie Aceton, Methylethylketon, Tetrahydrofuran, Dioxan, Chlorbenzol, Dimethylformamid oder Dimethylsulfoxid gegebenenfalls in Gegenwart einer anorganischen Base, z.B. Natriumcarbonat oder Kaliumhydroxid, oder einer teriären organischen Base, z.B. Triethylamin oder Pyridin, wobei letztere gleichzeitig auch als Lösungsmittel dienen können, und gegebenenfalls in Gegenwart eines Reaktionsbeschleunigers wie eines Kupfersalzes, eines entsprechenden Amin-hydrohalogenids oder Alkalihalogenids bei Temperaturen zwischen 0 und 150 ° C, vorzugsweise jedoch bei Temperaturen zwischen 20 und 120 ° C, durchgeführt. Die Umsetzung kann jedoch auch ohne Lösungsmittel oder in einem Überschuß der eingesetzten Verbindung der allgemeinen Formel III durchgeführt werden.

b) Zur Herstellung von Verbindungen der allgemeinen Formel I, in der $R_a$ eine >CH-($R_2NR_3$)-Gruppe darstellt:

Reduktive Aminierung einer Verbindung der allgemeinen Formel

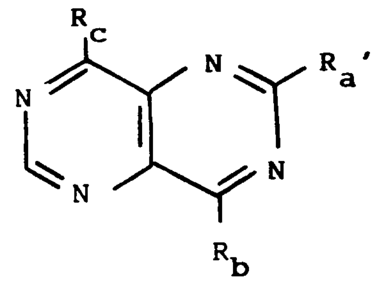

, (IV)

in der

$R_b$ und $R_c$ wie eingangs definiert sind und

$R_{a'}$ eine Pyrrolidino-, Piperidino- oder Hexamethyleniminogruppe darstellt, wobei die Methylengruppe der Pyrrolidinogruppe in 3-Stellung oder der Piperidino- oder Hexamethyleniminogruppe in 3- oder 4-Stellung durch eine >CO-Gruppe ersetzt ist, mit einem Amin der allgemeinen Formel

$H - R_2NR_3$ , (V)

in der

$R_2$ und $R_3$ wie eingangs definiert sind.

Die reduktive Aminierung wird zweckmäßigerweise in einem Lösungsmittel wie Methanol, Ethanol, Tetrahydrofuran oder Dioxan vorzugsweise in Gegenwart eines komplexen Metallhydrids wie Natriumborhydrid, Lithiumborhydrid oder Natriumcyanborhydrid zweckmäßigerweise bei Raumtemperatur oder in Gegenwart eines Hydrierungskatalysators, z.B. mit Wasserstoff in Gegenwart von Palladium/Kohle, bei einem Wasserstoffdruck 1 bis 5 bar durchgeführt.

Erhält man erfindungsgemäß eine Verbindung der allgemeinen Formel I, die eine $>(R_4OCOR_5)$-Gruppe enthält, so kann diese mittels Hydrolyse in eine entsprechende Carbonylverbindung der allgemeinen Formel I übergeführt werden, oder

eine Verbindung der allgemeinen Formel I, die eine Alkoxycarbonylgruppe enthält, so kann diese mittels Hydrolyse in eine entsprechende Carboxyverbindung der allgemeinen Formel I übergeführt werden, oder

erhält man eine Verbindung der allgemeinen Formel I, die eine Carboxygruppe enthält, so kann diese mittels Veresterung oder Amidierung in eine entsprechende Verbindung der allgemeinen Formel I, die eine Alkoxycarbonyl-, Aminocarbonyl-, Alkylaminocarbonyl- oder Dialkylaminocarbonylgruppe enthält, übergeführt werden, oder

eine Verbindung der allgemeinen Formel I, die eine Thiomorpholinogruppe enthält, so kann diese mittels Oxidation in eine entsprechende 1-Oxido-thiomorpholinoverbindung der allgemeinen Formel I übergeführt werden, oder

eine Verbindung der allgemeinen Formel I, die eine Thiomorpholino- oder 1-Oxido-thiomorpholinogruppe enthält, so kann diese mittels Oxidation in eine entsprechende 1,1-Dioxido-thiomorpholinoverbindung der allgemeinen Formel I übergeführt werden.

Die gegebenenfalls anschließende Hydrolyse wird vorzugsweise in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches durchgeführt.

Die gegebenenfalls anschließende Veresterung und/oder Amidierung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan oder besonders vorteilhaft in einem entsprechenden Alkohol oder Amin als Lösungsmittel gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol und gegebenenfalls zusätzlich in Gegenwart von 4-Dimethylaminopyridin, N,N'-Carbonyldiimidazol oder Triphenylphosphin/Tetrachlorkohlenstoff, zweckmäßigerweise bei Temperaturen zwischen 0 und 150°C, vorzugsweise bei Temperaturen zwischen 0 und 80°C, durchgeführt.

Die nachträgliche Oxidation wird vorzugsweise in einem Lösungsmittel oder Lösungsmittelgemisch, z.B. in Wasser, Wasser/Pyridin, Aceton, Methylenchlorid, Eisessig, Eisessig/Acetanhydrid, verdünnter Schwefelsäure oder Trifluoressigsäure, je nach dem verwendeten Oxidationsmittel zweckmäßigerweise bei Temperaturen zwischen -80 und 100 ° C durchgeführt.

Zur Herstellung einer entsprechenden 1-Oxidoverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20 ° C, mit einer Persäure wie Perameisensäure in Eisessig oder Trifluoressigsäure bei 0 bis 50 ° C oder mit m-Chlorperbenzoesäure in Methylenchlorid oder Chloroform bei -20 bis 60 ° C, mit Natriummetaperjodat in wäßrigem Methanol oder Ethanol bei -15 bis 25 ° C, mit Brom in Eisessig oder wäßriger Essigsäure gegebenenfalls in Gegenwart einer schwachen Base wie Natriumacetat, mit N-Brom-succinimid in Ethanol, mit tert.Butyl-hypochlorit in Methanol bei -80 bis -30 ° C, mit Jodbenzodichlorid in wäßrigem Pyridin bei 0 bis 50 ° C, mit Salpetersäure in Eisessig bei 0 bis 20 ° C, mit Chromsäure in Eisessig oder in Aceton bei 0 bis 20 ° C und mit Sulfurylchlorid in Methylenchlorid bei -70 ° C, der hierbei erhaltene Thioether-Chlor-Komplex wird zweckmäßigerweise mit wäßrigem Ethanol hydrolysiert.

Zur Herstellung einer entsprechenden 1,1-Dioxidoverbindung der allgemeinen Formel I wird die Oxidation ausgehend von einer entsprechenden 1-Oxidoverbindung zweckmäßigerweise mit einem oder mehr Äquivalenten des verwendeten Oxidationsmittels oder ausgehend von einer entsprechenden Thioverbindung zweckmäßigerweise mit zwei oder mehr Äquivalenten des verwendeten Oxidationsmittels durchgeführt, z.B. mit Wasserstoffperoxid in Eisessig/Acetanhydrid, Trifluoressigsäure oder in Ameisensäure bei 20 bis 100 ° C, mit einer Persäure wie Perameisensäure oder m-Chlorperbenzoesäure in Eisessig, Trifluoressigsäure, Methylenchlorid oder Chloroform bei Temperaturen zwischen 0 und 60 ° C, mit Salpetersäure in Eisessig bei 0 bis 20 ° C, mit Chromsäure oder Kaliumpermanganat in Eisessig, Wasser/Schwefelsäure oder in Aceton bei 0 bis 20 ° C.

Bei den vorstehend beschriebenen Umsetzungen können gegebenenfalls vorhandene reaktive Gruppen wie Hydroxy-, Amino-, Alkylamino- oder Carbonylgruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.Butyl-, Benzyl- oder Tetrahydropyranylgruppe,
als Schutzrest für eine Amino-, Alkylamino- oder Iminogruppe die Acetyl-, Benzoyl-, Ethoxycarbonyl- oder Benzylgruppe und
als Schutzrest für eine Carbonylgruppe die 1,3-Dioxan- oder 1,3-Dioxolangruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 ° C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 ° C, vorzugsweise jedoch bei Raumtemperatur, und einem Wasserstoffdruck von 1 bis 7 bar, vorzugsweise jedoch von 3 bis 5 bar.

Desweiteren können die erhaltenen Verbindungen der allgemeinen Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der allgemeinen Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der allgemeinen Formeln II bis V sind teilweise literaturbekannt oder man erhält diese nach literaturbekannten Verfahren.

So erhält man beispielsweise die als Ausgangsstoffe verwendeten Verbindungen der allgemeinen Formeln II und IV durch stufenweisen Ersatz der Chloratome des 2,4,8-Trichlor-pyrimido[5,4-d] pyrimidins (siehe DE-C-1 116 676).

Wie bereits eingangs erwähnt, weisen die neuen Verbindungen der allgemeinen Formel I wertvolle Eigenschaften auf. So stellen die Verbindungen der allgemeinen Formel I, in der $R_a$ eine Pyrrolidino-, Piperidino- oder Hexamethyleniminogruppe, wobei eine Methylengruppe in einer Pyrrolidinogruppe in 3-Stellung oder in einer Piperidino- und Hexamethyleniminogruppe in 3- oder 4-Stellung jeweils durch eine >CO- oder >($R_4OCOR_5$)-Gruppe ersetzt ist, wertvolle Zwischenprodukte zur Herstellung der übrigen Verbindungen der allgemeinen Formel I dar, welche wertvolle pharmakologische Eigenschaften aufweisen, insbesondere eine sensibilisierende Wirkung auf resistente Tumore bei der Chemotherapie.

Beispielsweise wurde die Sensibilisierung durch die Verbindungen

A = 2-[4-(N,N-Dimethylamino)-piperidino]-8-(N-benzyl-N-methyl-amino)-4-morpholino-pyrimido[5,4-d]pyrimidin,

B = 2-[4-(N-Methoxycarbonyl-amino)-piperidino]-8-(N-benzyl-N-methyl-amino)-4-morpholino-pyrimido[5,4-d]pyrimidin,

C = 2-[4-(N,N-Dimethylamino)-piperidino]-8-(N-benzyl-N-methyl-amino)-4-piperidino-pyrimido[5,4-d]-pyrimidin,

D = 8-(N-Benzyl-N-methyl-amino)-2-[4-(piperidino)-piperidin-1-yl]-4-morpholino-pyrimido[5,4-d]-pyrimidin,

E = 2-[4-(N,N-Dimethylaminomethyl)-piperidino]-8-[N-(naphth-1-yl-methyl)-N-methyl-amino]-4-morpholino-pyrimido-[5,4-d]pyrimidin,

F = 4-(N-Benzyl-N-methyl-amino)-2-[4-(N,N-dimethylamino)-piperidino]-8-morpholino-pyrimido[5,4-d]pyrimidin,

G = 2-[4-(N,N-Dimethylamino)-piperidino]-8-(6,7-dimethoxy-1,2,3,4-tetrahydro-isochinolin-2-yl)-4-morpholino-pyrimido[5,4-d]pyrimidin,

H = 2-[4-(N,N-Dimethylamino)-piperidino]-8-[N-(naphth-1-yl-methyl)-N-methyl-amino]-4-morpholino-pyrimido[5,4-d]pyrimidin,

I = 2-[4-(2,2-Diphenylethylamino)-piperidino]-4-(N-benzyl-N-methyl-amino)-8-morpholino-pyrimido-[5,4-d]pyrimidin,

J = 2-[4-(N,N-Dimethylamino)-piperidino]-8-[N-benzyl-N-(3-benzoylaminopropyl)-amino]-4-morpholino-pyrimido-[5,4-d]pyrimidin und

K = 2-[4-(N,N-Dimethylamino)-piperidino]-4-[N-(naphth-1-yl-methyl)-N-methyl-amino]-8-morpholino-pyrimido[5,4-d]pyrimidin

am Beispiel von gegen Adriamycin resistenten Zellen wie folgt geprüft:

Proliferierende, adriamycinresistente S 180 Maus Sarcomazellen werden in Anwesenheit unterschiedlicher Konzentrationen Testsubstanzen für sechs Tage kultiviert. Zytotoxisch oder zytostatisch wirkende Konzentrationen der Testsubstanzen werden durch vermindertes Zellwachstum oder durch das Absterben der Zellen angezeigt. Endpunkt des Assays ist die Zahl der lebenden Zellen pro Kultur, die indirekt ermittelt wird, indem die Eigenschaft vitaler Zellen, den Farbstoff MTT [= 3-(4,5-Dimethylthiazol-2-yl)-2,5-diphenyltetrazoliumbromid] zum farbigen Formazan zu reduzieren, ausgenützt wird. Als IC50 wird die Konzentration einer Testsubstanz bezeichnet, welche die Zahl der vitalen Zellen pro Kulturgefäß auf 50 % der unbehandelten Kontrolle reduziert. Die Testsubstanzen werden sowohl in Abwesenheit von Adriamycin als auch in Anwesenheit einer unter Kulturbedingungen nicht proliferationshemmend wirkenden Menge Adriamycin getestet. Pro Testsubstanz erhält man daher zwei IC50-Werte, einen in Anwesenheit (IC50 ADR), den anderen in Abwesenheit (IC50) von Adriamycin. Die Differenz der Zehnerlogarithmen der beiden IC50-Werte: $\Delta$ = lgIC50-lgIC50 ADR ist ein Maß für die Erhöhung der Zytotoxizität der Testsubstanz durch Adriamycin.

Versuchungsdurchführung:

Exponentiell wachsende, adriamycinresistente oder adriamycinsensitive S 180-Zellen werden in 96-Loch Flachboden-Mikrotiterplatten zu 2000 Zellen pro Loch in 100 µl Wachstumsmedium (RPMI-1640, das 10 % fötales Rinderserum enthält) ausplattiert. Die Kulturplatten werden im Brutschrank bei 37°C, 5 % $CO_2$ und 100 % relativer Luftfeuchtigkeit inkubiert. Nach 24 Stunden werden pro Loch 50 µl Wachstumsmedium, das unterschiedliche Konzentrationen an Testsubstanz enthält, und 50 µl Wachstumsmedium mit oder ohne Adriamycin hinzugefügt. Im Anschluß an eine weitere sechstägige Kultivierung werden 50 µl Tetrazoliumsalzlösung [5 mg 3-(4,5-Dimethylthiazol-2-yl)-2,5-diphenyltetrazoliumbromid pro ml Phosphat-gepufferter Salinelösung, vor Gebrauch 1:5 (v/v) mit RPMI-1640 verdünnt] in jedes Loch pipettiert. Nach vier Stunden Inkubation wird das Kulturmedium vorsichtig abgesaugt und intrazellulär gebildetes Formazan durch 150 µl Dimethylsulfoxid pro Loch solubilisiert. Die Platten werden kurz geschüttelt und die optische Dichte bei 570 nm mit einem Photometer wie einem Dynatech MR-600-Gerät gemessen. Die Bildung des farbigen

Formazans durch Reduktion des Tetrazoliumsalzes ist proportional der Zahl der lebenden Zellen. Die Mittelwerte von Dreifach-Bestimmungen wurden bei der Berechnung der IC50-Werte verwendet (Verdünnungsstufe: 1:2).

| Substanz | IC50 [µg/ml] Adriamycin in ng/ml | | $lg \frac{IC50}{IC50\ 100\ ng\ Adriamycin}$ |
|---|---|---|---|
| | 0 | 100 | |
| A | 1 | 0,3 | 0,53 |
| B | 10 | 1,0 | 1,00 |
| C | 2 | 0,3 | 0,82 |
| D | 3 | 0,3 | 1,00 |
| E | 3 | 0,1 | 1,48 |
| F | 3 | 0,3 | 1,00 |
| G | 3 | 0,1 | 1,48 |
| H | 3 | 0,1 | 1,48 |
| I | 10 | 0,3 | 1,52 |
| J | 3 | 0,1 | 1,48 |
| K | 3 | 0,1 | 1,48 |

Die Pyrimido[5,4-d]pyrimidine der obigen allgemeinen Formel I weisen somit eine ausgeprägte Sensibilisierung auf adriamycinresistente Sarcomazellen auf und eignen sich daher gegebenenfalls in Kombination mit einem Chemotherapeuticum zur Sensibilisierung von bei der Chemotherapie resistenten Tumoren, das heißt, deren Resistenz gegenüber einer diesbezüglichen Chemotherapie wird aufgehoben und somit die Remission der gegenüber diesen Substanzen resistenten Tumoren bewirkt. Als Chemotherapeutica kommen beispielsweise Vinca Alkaloide wie Vinblastin, Vincristin oder Vindesin, Epipodophyllotoxine wie VP16 oder Antracyllin-Antibioticawie Daunorubicin, Doxorubicin oder Bleomycin, Colchicin, Mitoxantron, Taxol, Taxotere, Mithramycin oder Mitomycin in Betracht.

Die vorstehend erwähnten neuen Pyrimido[5,4-d]pyrimidine der Formel I verhindern also zusammen mit einem Chemotherapeuticum, daß therapieresistente Tumorzellsubpopulationen die Therapie überleben und zum Rezidiv führen können.

Die Pyrimido[5,4-d]pyrimidine der allgemeinen Formel I sind gut verträgliche, da beispielsweise bei der Applikation der Verbindung G in einer Dosis von 100 mg/kg i.v. an der Maus keine toxischen Nebenwirkungen beobachtet werden konnten.

Die Application der erfindungsgemäßen neuen Pyrimido[5,4-d]pyrimidine erfolgt vorzugsweise getrennt und zweckmäßig bereits einige Tage, z.B. ein oder zwei Tage, vor der Application eines Chemotherapeuticums oder kombiniert mit einem in der üblichen Dosis applizierten Chemotherapeuticum; die Dosis des eingesetzten Pyrimido[5,4-d]pyrimidins liegt hierbei zwischen 1 und 50 mg/kg Körpergewicht pro Tag, vorzugsweise zwischen 3 bis 20 mg/kg Körpergewicht pro Tag, verteilt auf 1 bis 4 Einzeldosen. Die Applikation erfolgt jedoch vorzugsweise per Infusion.

Kombiniert mit einem entsprechenden Chemotherapeuticum kommt hierbei eine intravenöse Darreichungsform wie Ampullen und bei einer getrennten Application eine vorherige oder parallele Darreichungsform von Tabletten, Dragées, Suspensionen, Säfte, Kapseln oder Zäpfchen in Betracht.

Beispielsweise erfolgt auf Grund des literaturbekannten Applicationsschemas der bei der Chemotherapie von neoplastischen Erkrankungen eingesetzten Naturprodukte (siehe Goodman and Gilman's, The Pharmacoligical Basis of Therapeutics, Macmillan Publishing Company, New York, 7. Auflage, Seiten 1240-1247 und 1277-1289 (1985)) die erste Application eines Pyrimido[5,4-d]-pyrimidins der Formel I oder dessen physiologisch verträglichem Säureadditionssalz zweckmäßigerweise vor dem oder zusammen mit

dem verwendeten Chemotherapeuticum bzw. vor oder mit einer Kombination mehrerer Chemotherapeutica (siehe DeVita et al. in "Cancer, Principles & Practice of Oncology", 2nd Edition, J. B. Lippincott Company Philadelphia).

Die übrigen Applikationen eines Pyrimido[5,4-d]pyrimidins der Formel I oder dessen physiologisch verträglichen Säureadditionssalzes können den Umständen entsprechend peroral oder ebenfalls intravenös erfolgen.

Eine erfindungsgemäß für die i.v.-Application geeignete Kombination enthält somit zweckmäßigerweise 1 bis 25 mg/kg, vorzugsweise 1 bis 20 mg/kg Körpergewicht, eines Pyrimido[5,4-d]pyrimidins der Formel I oder dessen physiologisch verträgliches Säureadditionssalz und ein geeignetes Chemotherapeuticum oder eine Kombination verschiedener geeigneter Chemotherapeutica.

Die nachfolgenden Beispiele sollen die Erfindung näher erläutern:

Herstellung der Ausgangsverbindungen:

Beispiel I

2,8-Dichlor-4-morpholino-pyrimido[5,4-d]pyrimidin

9 g 2,4,8-Trichlor-pyrimido[5,4-d]pyrimidin werden in 70 ml Aceton suspendiert und mit 3,3 g Morpholin in 10 ml Aceton bei 0 °C versetzt. Die Reaktionsmischung wird eine Stunde unter Eiskühlung gerührt und dann auf 100 ml Wasser gegossen. Der ausgefallene Niederschlag wird abgesaugt, mit Wasser gewaschen und im Exsikator getrocknet.

Ausbeute: 9,5 g (81 % der Theorie).
$R_f$-Wert: 0,96 (Kieselgel; Cyclohexan/Essigsäureethylester = 2:1)

Analog werden hergestellt:

2,8-Dichlor-4-diethylamino-pyrimido[5,4-d]pyrimidin
$R_f$-Wert: 0,45 (Kieselgel; Methylenchlorid)
2,8-Dichlor-4-[N-(napht-1-yl-methyl)-N-methyl-amino]-pyrimido[5,4-d]pyrimidin
2,8-Dichlor-4-(1,2,3,4-tetrahydro-isochinolin-2-yl)-pyrimido[5,4-d]pyrimidin
2,8-Dichlor-4-piperidino-pyrimido[5,4-d]pyrimidin
$R_f$-Wert: 0,66 (Kieselgel; Cyclohexan/Essigester = 2:1)
2,4-Dichlor-4-(N-methyl-benzylamino)-pyrimido[5,4-d]pyrimidin
$R_f$-Wert: 0,5 (Kieselgel; Cyclohexan/Essigsäureethylester = 2:1)

Beispiel II

2-Chlor-8-(6,7-dimethoxy-1,2,3,4-tetrahydro-isochinolin-2-yl)-4-morpholino-pyrimido[5,4-d]pyrimidin

1 g (3,2 mMol) 2,8-Dichlor-4-morpholino-pyrimido[5,4-d]pyrimidin werden in 15 ml Aceton gelöst und bei Raumtemperatur mit 1,9 g (8,1 mMol) 6,7-Dimethoxy-1,2,3,4-tetrahydro-isochinolin in wenig Aceton versetzt. Nach 3 Stunden Rühren wird auf 20 ml Wasser gegossen und der Niederschlag abgesaugt und getrocknet.

Ausbeute: 1,2 g (83 % der Theorie),
$R_f$-Wert: 0,30 (Kieselgel; Cyclohexan/Essigester = 2:1)

Analog werden hergestellt:

8-(N-Benzyl-N-methyl-amino)-2-chlor-4-morpholino-pyrimido[5,4-d]pyrimidin
$R_f$-Wert: 0,60 (Kieselgel; Cyclohexan/Essigester = 2:1)
2-Chlor-4-morpholino-8-(1,2,3,4-tetrahydro-isochinolin-2-yl)-pyrimido[5,4-d]pyrimidin
$R_f$-Wert: 0,50 (Kieselgel; Cyclohexan/Essigester = 2:1)
2-Chlor-8-[N-(4-methoxy-benzyl)-N-methyl-amino]-4-morpholino-pyrimido[5,4-d]pyrimidin
Schmelzpunkt: 157-158 °C
2-Chlor-8-[N-(4-methyl-benzyl)-N-methyl-amino]-4-morpholino-pyrimido[5,4-d]pyrimidin
Schmelzpunkt: 156-157 °C
2-Chlor-8-[N-(4-chlor-benzyl)-N-methyl-amino]-4-morpholino-pyrimido[5,4-d]pyrimidin

Schmelzpunkt: 149-151 °C

2-Chlor-8-(N-cyclohexylmethyl-N-methyl-amino)-4-morpholino-pyrimido[5,4-d]pyrimidin

Schmelzpunkt: 98-100 °C

2-Chlor-4-morpholino-8-[N-(naphth-2-yl-methyl)-N-methyl-amino]-pyrimido[5,4-d]pyrimidin

Schmelzpunkt: 132-134 °C

2-Chlor-8-[N-(4-methoxy-naphth-1-yl-methyl)-N-methyl-amino]-4-morpholino-pyrimido[5,4-d]pyrimidin

Schmelzpunkt: 131-135 °C

2-Chlor-8-[N-(4-N,N-dimethylamino-naphth-1-yl-methyl)-N-methyl-amino]-4-morpholino-pyrimido[5,4-d]-pyrimidin

Schmelzpunkt: 145-148 °C

2-Chlor-8-[N-(naphth-1-yl-methyl)-N-methyl-amino]-4-piperidin-yl-pyrimido[5,4-d]pyrimidin

$R_f$-Wert: 0,62 (Kieselgel; Cyclohexan/Essigester = 2:1)

2-Chlor-8-(N-benzyl-N-ethyl-amino)-4-morpholino-pyrimido-[5,4-d]pyrimidin

Schmelzpunkt: 108-110 °C

2-Chlor-4-morpholino-8-(3-phenylpropylamino)-pyrimido[5,4-d]pyrimidin

Schmelzpunkt: 100-102 °C

2-Chlor-4-morpholino-8-(2-phenylethylamino)-pyrimido[5,4-d]pyrimidin

Schmelzpunkt: 141-143 °C

2-Chlor-8-(cyclohexylmethylamino)-4-morpholino-pyrimido[5,4-d]pyrimidin

Schmelzpunkt: 125-127 °C

8-Benzylamino-2-chlor-4-morpholino-pyrimido[5,4-d]pyrimidin

Schmelzpunkt: 148-151 °C

2-Chlor-8-(4-methyl-benzylamino)-4-morpholino-pyrimido[5,4-d]pyrimidin

Schmelzpunkt: 180-182 °C

2-Chlor-8-(4-methoxy-benzylamino)-4-morpholino-pyrimido[5,4-d]pyrimidin

Schmelzpunkt: 156-158 °C

8-(N-Benzyl-N-methyl-amino)-2-chlor-4-diethylamino-pyrimido[5,4-d]pyrimidin

Öl, $R_f$-Wert: 0,52 (Kieselgel; Methylenchlorid)

2-Chlor-8-[N-(naphth-1-yl-methyl)-N-methyl-amino]-4-morpholino-pyrimido[5,4-d]pyrimidin

Öl, $R_f$-Wert: 0,42 (Kieselgel; Cyclohexan/Essigester = 2:1)

2-Chlor-4-morpholino-8-(1,2,3,4-tetrahydro-isochinolin-2-yl)-pyrimido[5,4-d]pyrimidin

Öl, $R_f$-Wert: 0,40 (Kieselgel; Cyclohexan/Essigester = 2:1)

8-(N-Benzyl-N-methyl-amino)-2-chlor-4-piperidino-pyrimido[5,4-d]pyrimidin

Öl, $R_f$-Wert: 0,79 (Kieselgel; Cyclohexan/Essigester = 2:1)

8-Benzyloxy-2-chlor-4-morpholino-pyrimido[5,4-d]pyrimidin

$R_f$-Wert: 0,45 (Kieselgel; Cyclohexan/Essigester = 2:1)

2-Chlor-4,8-bis(morpholino)-pyrimido[5,4-d]pyrimidin

$R_f$-Wert: 0,16 (Kieselgel; Methylenchlorid/Methanol = 39:1)

8-(N-Benzyl-N-ethyloxycarbonylmethyl-amino)-2-chlor-4-piperidino-pyrimido[5,4-d]pyrimidin

Aus N-Benzylglydinethylester und 2,8-Dichlor-4-morpholino-pyrimido[5,4-d]pyrimidin

Schmelzpunkt: 87-91 °C

8-[N-Benzyl-N-(2-cyanoethyl)-amino]-2-chlor-4-morpholino-pyrimido[5,4-d]pyrimidin

Aus 3-Benzylaminopropionitril und 2,8-Dichlor-4-morpholino-pyrimido[5,4-d]pyrimidin

Schmelzpunkt: 108-112 °C

8-[N-(Anthracen-9-yl)-N-methyl-amino]-2-chlor-4-morpholino-pyrimido[5,4-d]pyrimidin

$R_f$-Wert: 0,91 (Kieselgel; Methylenchlorid/Methanol = 9:1)

8-(N-Benzyl-N-isopropyl-amino)-2-chlor-4-morpholino-pyrimido[5,4-d]pyrimidin

$R_f$-Wert: 0,64 (Kieselgel; Cyclohexan/Essigester = 2:1)

2-Chlor-8-dibenzylamino-4-morpholino-pyrimido[5,4-d]pyrimidin

$R_f$-Wert: 0,62 (Kieselgel; Cyclohexan/Essigester = 2:1)

2-Chlor-4-(N-benzyl-N-methyl-amino)-8-morpholino-pyrimido-[5,4-d]pyrimidin

$R_f$-Wert: 0,52 (Kieselgel; Cyclohexan/Essigester = 2:1)

2-Chlor-8-[2-(3,4-dimethoxyphenyl)ethyl-methylamino]-4-morpholino-pyrimido[5,4-d]pyrimidin

$R_f$-Wert: 0,71 (Kieselgel; Methylenchlorid/Methanol = 9:1)

2-Chlor-8-(isoindolin-2-yl)-4-morpholino-pyrimido[5,4-d]pyrimidin

$R_f$-Wert: 0,9 (Kieselgel; Methylenchlorid/Methanol = 9:1)

2-Chlor-8-(5-methoxy-1,2,3,4-tetrahydroisochinolin-2-yl)-4-morpholino-pyrimido[5,4-d]pyrimidin

$R_f$-Wert: 0,66 (Kieselgel; Cyclohexan/Essigsäureethylester = 2:1)

2-Chlor-8-benzylthio-4-morpholino-pyrimido[5,4-d]pyrimidin
$R_f$-Wert: 0,72 (Kieselgel; Methanol/Methylenchlorid = 50:1)
2-Chlor-8-(5-acetylamino-1,2,3,4-tetrahydroisochinolin-2-yl)-4-morpholino-pyrimido[5,4-d]pyrimidin
$R_f$-Wert: 0,55 (Kieselgel; Methylenchlorid/Methanol = 9:1)
2-Chlor-8-(6,7-dihydroxy-1,2,3,4-tetrahydroisochinolin-2-yl)-4-morpholino-pyrimido[5,4-d]pyrimidin
Schmelzpunkt: 194-197 °C
2-Chlor-8-(6,7-methylendioxy-1,2,3,4-tetrahydroisochinolin-2-yl)-4-morpholino-pyrimido[5,4-d]pyrimidin
Hergestellt aus 2-Chlor-8-(6,7-dihydroxy-1,2,3,4-tetrahydroisochinolin-2-yl)-4-morpholino-pyrimido[5,4-d]-pyrimidin durch Alkylierung mit Bromchlormethan in Dimethylformamid.
Schmelzpunkt: 187-189 °C

Herstellung der Endprodukte:

Beispiel 1

2-[4-(N,N-Dimethylamino)-piperidino]-8-(6,7-dimethoxy-1,2,3,4-tetrahydro-isochinolin-2-yl)-4-morpholino-pyrimido[5,4-d]pyrimidin

1,2 g (2,7 mMol) 2-Chlor-8-(6,7-dimethoxy-1,2,3,4-tetrahydro-isochinolin-2-yl)-4-morpholino-pyrimido-[5,4-d]pyrimidin werden in 20 ml Dioxan gelöst, mit 1,4 g (11 mMol) 4-Dimethylamino-piperidin versetzt und 4 Stunden am Rückfluß gekocht. Dann wird die Lösung auf Wasser gegossen und das Produkt mit Essigester extrahiert. Die organische Phase wird über Natriumsulfat getrocknet und einrotiert. Der Rückstand wird durch Chromatographie über eine Kieselgelsäule mit Methylenchlorid/Methanol/Ammoniak = 30:1:0,1 gereinigt.
Ausbeute: 0,5 g (36 % der Theorie),
Schmelzpunkt: 125 °C

| $C_{28}H_{38}N_8O_3$ (534,67) | | | | | | |
|---|---|---|---|---|---|---|
| Ber.:<br>Gef.: | C | 62,90<br>62,82 | H | 7,16<br>7,26 | N | 20,96<br>20,72 |

Analog werden hergestellt:

(1) 2-[4-(N,N-Dimethylamino)-piperidino]-8-(N-benzyl-N-methylamino)-4-morpholino-pyrimido[5,4-d]-pyrimidin
Ausbeute: 50 % der Theorie,
Schmelzpunkt: 98 °C

| $C_{25}H_{34}N_8O$ (462,60) | | | | | | |
|---|---|---|---|---|---|---|
| Ber.:<br>Gef.: | C | 64,91<br>64,77 | H | 7,41<br>7,52 | N | 24,23<br>24,18 |

(2) 2-[4-(N,N-Dimethylamino)-piperidino]-8-(1,2,3,4-tetrahydro-isochinolin-2-yl)-4-morpholino-pyrimido[5,4-d]pyrimidin-oxalat
Ausbeute: 20 % der Theorie,
Schmelzpunkt: 145 °C

| $C_{26}H_{34}N_8O \times (COOH)_2$ (564,65) | | | | | | |
|---|---|---|---|---|---|---|
| Ber.:<br>Gef.: | C | 59,56<br>59,57 | H | 6,43<br>6,69 | N | 19,84<br>19,68 |

(3) 2-[4-(N,N-Dimethylamino)-piperidino]-8-[N-(4-methoxy-benzyl)-N-methyl-amino]-4-morpholino-pyrimido[5,4-d]pyrimidin

Ausbeute: 33,8 % der Theorie,
Schmelzpunkt: 109-110 ° C

| $C_{26}H_{36}N_8O_2$ (492,63) | | | | | | |
|---|---|---|---|---|---|---|
| Ber.:<br>Gef.: | C | 63,39<br>63,14 | H | 7,37<br>7,34 | N | 22,75<br>22,44 |

(4) 2-[4-(N,N-Dimethylamino)-piperidino]-8-[N-(4-methyl-benzyl)-N-methyl-amino]-4-morpholino-pyrimido-[5,4-d]pyrimidin
Ausbeute: 33 % der Theorie,
Schmelzpunkt: 234-235 ° C

| $C_{26}H_{36}N_8O$ x 0,5 $H_2O$ (485,64) | | | | | | |
|---|---|---|---|---|---|---|
| Ber.:<br>Gef.: | C | 64,30<br>64,12 | H | 7,68<br>7,65 | N | 23,08<br>22,83 |

(5) 2-[4-(N,N-Dimethylamino)-piperidino]-8-[N-(4-chlor-benzyl)-N-methyl-amino]-4-morpholino-pyrimido-[5,4-d]pyrimidin
Ausbeute: 51,7 % der Theorie,
Schmelzpunkt: 215-220 ° C

| $C_{25}H_{33}ClN_8O$ (497,05) | | | | | | |
|---|---|---|---|---|---|---|
| Ber.:<br>Gef.: | C | 60,41<br>60,42 | H | 6,69<br>6,69 | N | 22,55<br>22,33 |

(6) 2-[4-(N,N-Dimethylamino)-piperidino]-8-[N-(naphth-2-yl-methyl)-N-methyl-amino]-4-morpholino-pyrimido[5,4-d]pyrimidin
Ausbeute: 57,5 % der Theorie,
Schmelzpunkt: 114-117 ° C

| $C_{29}H_{36}N_8O$ (512,66) | | | | | | |
|---|---|---|---|---|---|---|
| Ber.:<br>Gef.: | C | 67,94<br>67,84 | H | 7,08<br>7,11 | N | 21,86<br>21,69 |

(7) 2-[4-(N,N-Dimethylamino)-piperidino]-8-[N-(4-methoxy-naphth-1-yl-methyl)-N-methyl-amino]-4-morpholino-pyrimido[5,4-d]pyrimidin
Ausbeute: 54,4 % der Theorie,
Schmelzpunkt: 130-132 ° C

| $C_{30}H_{38}N_8O_2$ x 0,5 $H_2O$ (551,70) | | | | | | |
|---|---|---|---|---|---|---|
| Ber.:<br>Gef.: | C | 65,31<br>65,32 | H | 7,12<br>7,01 | N | 20,31<br>20,19 |

(8) 2-[4-(N,N-Dimethylamino)-piperidinol-8-[N-(4-N,N-dimethylamino-naphth-1-yl-methyl)-N-methyl-amino]-4-morpholino-pyrimido[5,4-d]pyrimidin
Ausbeute: 35,2 % der Theorie,
Schmelzpunkt: 170 ° C

| $C_{31}H_{41}N_9O \times H_2O$ (573,74) | | | | | | |
|---|---|---|---|---|---|---|
| Ber.:<br>Gef.: | C | 64,90<br>64,57 | H | 7,55<br>7,47 | N | 21,97<br>21,87 |

(9) 2-[4-(N,N-Dimethylamino)-piperidino]-8-(N-benzyl-N-ethylamino)-4-morpholino-pyrimido[5,4-d]-pyrimidin

Ausbeute: 79 % der Theorie,

Öl, $R_f$-Wert: 0,25 (Kieselgel; Essigsäureethylester/Methanol/Ammoniak = 8:2:0,5)

| $C_{26}H_{36}N_8O$ (476,63) | | | | | | |
|---|---|---|---|---|---|---|
| Ber.:<br>Gef.: | C | 65,52<br>65,51 | H | 7,61<br>7,74 | N | 23,51<br>23,59 |

(10) 2-[4-(N,N-Dimethylamino)-piperidino]-8-[N-(naphth-1-yl-methyl)-N-methyl-amino]-4-morpholino-pyrimido[5,4-d]pyrimidin

Ausbeute: 67 % der Theorie,

Schmelzpunkt: 146-148 °C

| $C_{29}H_{36}N_8O$ (512,66) | | | | | | |
|---|---|---|---|---|---|---|
| Ber.:<br>Gef.: | C | 67,94<br>67,96 | H | 7,08<br>7,20 | N | 21,86<br>21,47 |

(11) 2-[4-(N,N-Dimethylamino)-piperidino]-8-(3-phenylpropylamino)-4-morpholino-pyrimido[5,4-d]pyrimidin

Ausbeute: 56 % der Theorie,

Schmelzpunkt: 82-84 °C

| $C_{26}H_{36}N_8O$ (476,63) | | | | | | |
|---|---|---|---|---|---|---|
| Ber.:<br>Gef.: | C | 65,52<br>64,92 | H | 7,61<br>7,65 | N | 23,51<br>22,72 |

(12) 2 [4-(N,N-Dimethylamino)-piperidino]-8-(2-phenylethylamino)-4-morpholino-pyrimido[5,4-d]pyrimidin

Ausbeute: 68 % der Theorie,

Schmelzpunkt: 115-117 °C

| $C_{25}H_{34}N_8O$ (462,60) | | | | | | |
|---|---|---|---|---|---|---|
| Ber.:<br>Gef.: | C | 64,91<br>64,60 | H | 7,41<br>7,36 | N | 24,22<br>24,16 |

(13) 2-[4-(N,N-Dimethylamino)-piperidino]-8-(cyclohexylmethylamino)-4-morpholino-pyrimido[5,4-d]-pyrimidin

Ausbeute: 58 % der Theorie,

Schmelzpunkt: 110-112 °C

| $C_{24}H_{38}N_8O$ (454,62) | | | | | | |
|---|---|---|---|---|---|---|
| Ber.:<br>Gef.: | C | 63,41<br>63,13 | H | 8,42<br>8,59 | N | 24,65<br>24,71 |

(14) 2-[4-(N,N-Dimethylamino)-piperidinol-8-benzylamino-4-morpholino-pyrimido[5,4-d]pyrimidin

Ausbeute: 58 % der Theorie,

Schmelzpunkt: 148-150 °C

| $C_{24}H_{32}N_8O$ (448,58) | | | | | | |
|---|---|---|---|---|---|---|
| Ber.: | C | 64,26 | H | 7,19 | N | 24,98 |
| Gef.: | | 63,89 | | 7,25 | | 23,89 |

(15) 2-[4-(N,N-Dimethylamino)-piperidino]-8-[N-(4-methyl-benzyl)-amino]-4-morpholino-pyrimido[5,4-d]-pyrimidin

Ausbeute: 47 % der Theorie,
Schmelzpunkt: 142-144 °C

| $C_{25}H_{34}N_8O$ (462,60) | | | | | | |
|---|---|---|---|---|---|---|
| Ber.: | C | 64,91 | H | 7,41 | N | 24,22 |
| Gef.: | | 64,86 | | 7,52 | | 24,32 |

(16) 2-[4-(N,N-Dimethylamino)-piperidino]-8-[N-(4-methoxy-benzyl)-amino]-4-morpholino-pyrimido[5,4-d]-pyrimidin

Ausbeute: 55 % der Theorie,
Schmelzpunkt: 124-126 °C

| $C_{25}H_{34}N_8O_2$ (478,60) | | | | | | |
|---|---|---|---|---|---|---|
| Ber.: | C | 62,74 | H | 7,16 | N | 23,41 |
| Gef.: | | 62,30 | | 7,30 | | 23,32 |

(17) 8-(N-Benzyl-N-methyl-amino)-2-[4-(piperidino)-piperidin-1-yl]-4-morpholino-pyrimido[5,4-d]pyrimidin

Aus 2-Chlor-8-(N-benzyl-N-methyl-amino)-4-morpholino-pyrimido[5,4-d]pyrimidin und 4-Piperidino-piperidin

Ausbeute: 54 % der Theorie,
Schmelzpunkt: 106 °C

| $C_{28}H_{38}N_8O$ (502,67) | | | | | | |
|---|---|---|---|---|---|---|
| Ber.: | C | 66,91 | H | 7,62 | N | 22,29 |
| Gef.: | | 67,02 | | 7,85 | | 22,33 |

(18) 8-(N-Benzyl-N-methyl-amino)-2-[4-(N,N-dimethylaminomethyl)-piperidino]-4-morpholino-pyrimido[5,4-d]pyrimidin

Aus 2-Chlor-8-(N-benzyl-N-methyl-amino)-4-morpholino-pyrimido[5,4-d]pyrimidin und 4-(N,N-Dimethylaminomethyl)-piperidin

Ausbeute: 30,6 % der Theorie,
Schmelzpunkt: 145-147 °C

| $C_{26}H_{36}N_8O$ (476,63) | | | | | | |
|---|---|---|---|---|---|---|
| Ber.: | C | 65,52 | H | 7,61 | N | 23,51 |
| Gef.: | | 65,24 | | 7,70 | | 23,59 |

(19) 8-(N-Benzyl-N-methyl-amino)-4-morpholino-2-(1-phenyl-1,3,8-triazaspiro[4,5]decan-4-on-8-yl)-pyrimido[5,4-d]pyrimidin

Aus 2-Chlor-8-(N-benzyl-N-methyl-amino)-4-morpholino-pyrimido[5,4-d]pyrimidin und 1-Phenyl-1,3,8-triazaspiro[4,5]-decan-4-on

Ausbeute: 47 % der Theorie,
Schmelzpunkt: 203-205 °C

| $C_{31}H_{35}N_9O_2$ (565,68) | | | | | | |
|---|---|---|---|---|---|---|
| Ber.:<br>Gef.: | C | 65,82<br>65,35 | H | 6,24<br>6,59 | N | 22,28<br>22,39 |

(20) 2-[4-(N,N-Dimethylamino)-piperidino]-8-(N-benzyl-N-methyl-amino)-4-diethylamino-pyrimido[5,4-d]-pyrimidin
Ausbeute: 23,8 % der Theorie,
Schmelzpunkt: 160-162 °C

| $C_{25}H_{36}N_8$ (448,62) | | | | | | |
|---|---|---|---|---|---|---|
| Ber.:<br>Gef.: | C | 66,93<br>66,85 | H | 8,09<br>8,05 | N | 24,98<br>25,30 |

(21) 2-[4-(N,N-Dimethylamino)-piperidino]-4-[N-(naphth-1-yl-methyl)-N-methyl-amino]-8-morpholino-pyrimido[5,4-d]pyrimidin
Ausbeute: 78 % der Theorie,
Schmelzpunkt: 124-126 °C

| $C_{29}H_{36}N_8O$ (512,66) | | | | | | |
|---|---|---|---|---|---|---|
| Ber.:<br>Gef.: | C | 67,94<br>68,00 | H | 7,08<br>7,21 | N | 21,86<br>21,76 |

(22) 2-[4-(N,N-Dimethylamino)-piperidino]-4-(1,2,3,4-tetrahydro-isochinolin-2-yl)-8-morpholino-pyrimido-[5,4-d]pyrimidin
Ausbeute: 81 % der Theorie,
Schmelzpunkt: 49-53 °C

| $C_{26}H_{34}N_8O$ (474,61) | | | | | | |
|---|---|---|---|---|---|---|
| Ber.:<br>Gef.: | C | 65,80<br>65,61 | H | 7,22<br>7,34 | N | 23,61<br>23,46 |

(23) 2-[4-(N,N-Dimethylamino)-piperidino]-8-(N-benzyl-N-methylamino)-4-piperidino-pyrimido[5,4-d]-pyrimidin
Ausbeute: 50 % der Theorie,

| $C_{26}H_{36}N_8$ (460,63) | | | | | | |
|---|---|---|---|---|---|---|
| Ber.:<br>Gef.: | C | 67,80<br>67,39 | H | 7,88<br>8,15 | N | 24,32<br>24,27 |

(24) 2-[4-(N,N-Dimethylamino)-piperidino]-4,8-bis(N-benzyl-amino)-pyrimido[5,4-d]pyrimidin
Ausbeute: 71 % der Theorie,
Schmelzpunkt: 143-145 °C

| $C_{27}H_{32}N_8$ (468,61) | | | | | | |
|---|---|---|---|---|---|---|
| Ber.:<br>Gef.: | C | 69,20<br>69,48 | H | 6,88<br>6,87 | N | 23,91<br>23,89 |

(25) 8-Benzyloxy-2-[4-(N,N-dimethylamino)-piperidino]-4-morpholino-pyrimido[5,4-d]pyrimidin
Ausbeute: 19 % der Theorie,

Schmelzpunkt: 186-188 °C

| $C_{24}H_{31}N_7O_2$ (449,56) | | | | | | |
|---|---|---|---|---|---|---|
| Ber.:<br>Gef.: | C | 64,12<br>64,23 | H | 6,95<br>6,96 | N | 21,81<br>21,69 |

(26) 2-[4-(N,N-Dimethylamino)-piperidino]-4,8-bis(morpholino)-pyrimido[5,4-d]pyrimidin

Ausbeute: 26 % der Theorie,
Schmelzpunkt: ab 140 °C

| $C_{21}H_{32}N_8O_2 \times H_2O$ (446,56) | | | | | | |
|---|---|---|---|---|---|---|
| Ber.:<br>Gef.: | C | 56,48<br>56,62 | H | 7,67<br>7,53 | N | 25,09<br>24,89 |

(27) 2-[4-(N,N-Dimethylamino)-piperidino]-8-(N-benzyl-N-ethoxycarbonylmethyl-amino)-4-morpholino-pyrimido[5,4-d]pyrimidin

Ausbeute: 97 % der Theorie,
Schmelzpunkt: 112-115 °C

| $C_{28}H_{38}N_8O_3$ (534,67) | | | | | | |
|---|---|---|---|---|---|---|
| Ber.:<br>Gef.: | C | 62,90<br>63,04 | H | 7,16<br>7,07 | N | 20,96<br>21,02 |

(28) 2-[4-(N,N-Dimethylamino)-piperidino]-8-[N-benzyl-N-(2-cyano-ethyl)-amino]-4-morpholino-pyrimido-[5,4-d]pyrimidin

Ausbeute: 89 % der Theorie,
Schmelzpunkt: 45-47 °C

| $C_{27}H_{35}N_9O$ (501,64) | | | | | | |
|---|---|---|---|---|---|---|
| Ber.:<br>Gef.: | C | 64,65<br>64,63 | H | 7,03<br>7,10 | N | 25,13<br>25,38 |

(29) 2 [4-(N,N-Dimethylaminomethyl)-piperidino]-8-[N-(naphth-1-yl-methyl)-N-methyl-amino]-4-morpholino-pyrimido[5,4-d]pyrimidin

Ausbeute: 21 % der Theorie,
Schmelzpunkt: 158 °C

| $C_{30}H_{38}N_8O$ (526,69) | | | | | | |
|---|---|---|---|---|---|---|
| Ber.:<br>Gef.: | C | 68,42<br>68,55 | H | 7,27<br>7,48 | N | 21,27<br>21,05 |

(30) 2-[4-(N,N-Dimethylaminomethyl)-piperidino]-8-[N-(naphth-1-yl-methyl)-N-methyl-amino]-4-piperidino-pyrimido[5,4-d]pyrimidin

Ausbeute: 41 % der Theorie,
Schmelzpunkt: 150-151 °C

| $C_{31}H_{40}N_8$ (524,72) | | | | | | |
|---|---|---|---|---|---|---|
| Ber.:<br>Gef.: | C | 70,96<br>71,04 | H | 7,68<br>7,44 | N | 21,36<br>21,68 |

(31) 4-(N-Benzyl-N-methyl-amino)-2-[4-(N,N-dimethylamino)-piperidino]-8-morpholino-pyrimido[5,4-d]-pyrimidin

Ausbeute: 47 % der Theorie,
Schmelzpunkt: 122 °C

| $C_{25}H_{34}N_8O$ (462,60) | | | | | | |
|---|---|---|---|---|---|---|
| Ber.:<br>Gef.: | C | 64,91<br>64,93 | H | 7,41<br>7,60 | N | 24,23<br>24,29 |

(32) 8-[N-(Anthracen-9-yl)-N-methyl-amino]-2-[4-(N,N-dimethylamino)-piperidino]-4-morpholino-pyrimido-[5,4-d]pyrimidin

Ausbeute: 18 % der Theorie,
Schmelzpunkt: 168 °C

| $C_{33}H_{38}N_8O$ (562,72) | | | | | | |
|---|---|---|---|---|---|---|
| Ber.:<br>Gef.: | C | 70,44<br>70,20 | H | 6,81<br>6,88 | N | 19,91<br>19,46 |

(33) 2-[4-(N-Methoxycarbonyl-amino)-piperidino]-8-(N-benzyl-N-methyl-amino)-4-morpholino-pyrimido-[5,4-d]pyrimidin

Ausbeute: 24 % der Theorie,
Schmelzpunkt: 110 °C

| $C_{26}H_{34}N_8O_3$ (506,61) | | | | | | |
|---|---|---|---|---|---|---|
| Ber.:<br>Gef.: | C | 61,64<br>61,47 | H | 6,76<br>6,91 | N | 22,12<br>22,17 |

(34) 2-[4-(N,N-Dimethylamino)-piperidino]-8-(N-cyclohexyl-methyl-N-methyl-amino)-4-morpholino-pyrimido[5,4-d]pyrimidin

Ausbeute: 19 % der Theorie,
Schmelzpunkt: 210-212 °C

| $C_{25}H_{40}N_8O$ x 0,5 $H_2O$ (477,66) | | | | | | |
|---|---|---|---|---|---|---|
| Ber.:<br>Gef.: | C | 62,86<br>62,76 | H | 8,65<br>8,41 | N | 23,46<br>23,53 |

(35) 2-[4-(N,N-Dimethylamino)-piperidino]-8-(N-benzyl-N-isopropyl-amino)-4-morpholino-pyrimido[5,4-d]-pyrimidin-oxalat

Ausbeute: 38 % der Theorie,
Schmelzpunkt: 122-125 °C

| $C_{27}H_{38}N_8O$ x $(COOH)_2$ (580,69) | | | | | | |
|---|---|---|---|---|---|---|
| Ber.:<br>Gef.: | C | 59,98<br>59,74 | H | 6,94<br>7,17 | N | 19,30<br>19,50 |

(36) 8-Dibenzylamino-2-[4-(N,N-dimethylamino)-piperidino]-4-morpholino-pyrimido[5,4-d]pyrimidin
Ausbeute: 47 % der Theorie,
Schmelzpunkt: 163 ° C

| $C_{31}H_{38}N_8O$ (538,70) | | | | | | |
|---|---|---|---|---|---|---|
| Ber.:<br>Gef.: | C | 69,12<br>69,01 | H | 7,11<br>7,06 | N | 20,80<br>20,34 |

(37) 2-(4-Pyrrolidino-piperidin-1-yl)-8-(N-benzyl-N-methyl-amino)-4-morpholino-pyrimido[5,4-d]pyrimidin-oxalat
Aus 2-Chlor-8-(N-benzyl-N-methyl-amino)-4-morpholino-pyrimido[5,4-d]pyrimidin und 4-Pyrrolidino-piperidin
Ausbeute: 57 % der Theorie,
Schmelzpunkt: 131-134 ° C

| $C_{27}H_{36}N_8O$ x $(COOH)_2$ (578,67) | | | | | | |
|---|---|---|---|---|---|---|
| Ber.:<br>Gef.: | C | 60,19<br>59,97 | H | 6,61<br>6,95 | N | 19,36<br>19,13 |

(38) 2-(8-Aza-1,4-dioxaspiro[4,5]decan-8-yl)-8-(N-benzyl-N-methyl-amino)-4-morpholino-pyrimido[5,4-d]-pyrimidin
Aus 2-Chlor-8-(N-benzyl-N-methyl-amino)-4-morpholino-pyrimido[5,4-d]pyrimidin und 8-Aza-1,4-dioxaspiro[4,5]decan
Ausbeute: 58 % der Theorie,
Schmelzpunkt: 143 ° C

| $C_{25}H_{31}N_7O_3$ (477,58) | | | | | | |
|---|---|---|---|---|---|---|
| Ber.:<br>Gef.: | C | 62,88<br>62,89 | H | 6,54<br>6,62 | N | 20,53<br>20,47 |

(39) 2-[4-(N,N-Dimethylamino-piperidino]-8-[2-(3,4-dimethoxyphenyl)-ethyl-methylamino]-4-morpholino-pyrimido[5,4-d]pyrimidin
Ausbeute: 16 % der Theorie,
Schmelzpunkt: 106 ° C

| $C_{28}H_{40}N_8O_3$ (536,68) | | | | | | |
|---|---|---|---|---|---|---|
| Ber.:<br>Gef.: | C | 62,66<br>62,63 | H | 7,51<br>7,33 | N | 20,88<br>20,92 |

(40) 2-[4-(N,N-Dimethylamino-piperidino]-8-(isoindolin-2-yl)-4-morpholino-pyrimido[5,4-d]pyrimidin
Ausbeute: 52 % der Theorie,
Schmelzpunkt: 195-197 ° C

| $C_{25}H_{32}N_8O$ (460,59) | | | | | | |
|---|---|---|---|---|---|---|
| Ber.:<br>Gef.: | C | 65,19<br>64,90 | H | 7,00<br>6,59 | N | 24,33<br>23,75 |

(41) 2-[4-(N,N-Dimethylaminomethyl)-piperidino]-8-(6,7-dimethoxy-1,2,3,4-tetrahydro-isochinolin-2-yl)-4-morpholino-pyrimido[5,4-d]pyrimidin-oxalat
Ausbeute: 16 % der Theorie,

$C_{29}H_{40}N_8O_3 \times 3H_2O \times (COOH)_2$ (692,77)

Schmelzpunkt: 67°C

| | | | | | | |
|---|---|---|---|---|---|---|
| Ber.: | C | 53,75 | H | 6,98 | N | 16,17 |
| Gef.: | | 53,91 | | 6,85 | | 15,54 |

(42) 2-[4-(N,N-Dimethylaminomethyl)-piperidino]-8-(5-methoxy-1,2,3,4-tetrahydro-isochinolin-2-yl)-4-morpholino-pyrimido[5,4-d]pyrimidin

Ausbeute: 32 % der Theorie,

Schmelzpunkt: 143 °C

| $C_{27}H_{36}N_8O_2$ (504,64) | | | | | | |
|---|---|---|---|---|---|---|
| Ber.:<br>Gef.: | C | 64,26<br>64,24 | H | 7,19<br>7,51 | N | 22,20<br>22,71 |

(43) 2-(8-Aza-1,4-dioxaspiro[4,5]decan-8-yl)-8-(6,7-dimethoxy-1,2,3,4-tetrahydroisochinolin-2-yl)-4-morpholino-pyrimido[5,4-d]pyrimidin

$R_f$-Wert: 0,65 (Kieselgel; Methylenchlorid/Methanol = 9:1)

(44) 2-[4-(N,N-Dimethylamino-piperidino]-8-benzylthio-4-morpholino-pyrimido[5,4-d]pyrimidin

Ausbeute: 30 % der Theorie,

Schmelzpunkt: 165 °C

| $C_{24}H_{31}N_7OS$ (465,63) | | | | | | |
|---|---|---|---|---|---|---|
| Ber.:<br>Gef.: | C | 61,91<br>62,03 | H | 6,71<br>6,71 | N | 21,06<br>20,79 |

(45) 2-[4-(N,N-Dimethylamino-piperidino]-8-(5-acetylamino-1,2,3,4-tetrahydroisochinolin-2-yl)-4-morpholino-pyrimido [5,4-d]pyrimidin

Ausbeute: 73 % der Theorie,

Schmelzpunkt: 195 °C

| $C_{28}H_{37}N_9O_2$ (531.67) | | | | | | |
|---|---|---|---|---|---|---|
| Ber.:<br>Gef.: | C | 63,26<br>63,12 | H | 7,01<br>6,95 | N | 23,71<br>23,94 |

(46) 2-(4-[N,N-Dimethylamino-piperidino]-8-(6,7-dihydroxy-1,2,3,4-tetrahydroisochinolin-2-yl)-4-morpholino-pyrimido [5,4-d]pyrimidin

Ausbeute: 57 % der Theorie,

Schmelzpunkt: 194-197 °C

| $C_{26}H_{34}N_8O_3$ (506,61) | | | | | | |
|---|---|---|---|---|---|---|
| Ber.:<br>Gef.: | C | 61,64<br>60,86 | H | 6,76<br>6,93 | N | 22,12<br>21,95 |

(47) 2-[4-(N,N-Dimethylamino-piperidino]-8-(6,7-methylendioxy-1,2,3,4-tetrahydroisochinolin-2-yl)-4-morpholino-pyrimido[5,4-d]pyrimidin

Ausbeute: 91 % der Theorie,

Schmelzpunkt: 99-101 °C

| $C_{27}H_{34}N_8O_3$ (518,62) | | | | | | |
|---|---|---|---|---|---|---|
| Ber.:<br>Gef.: | C | 62,53<br>62,05 | H | 6,61<br>6,63 | N | 21,61<br>21,19 |

(48) 2-[4-(N,N-Dimethylamino)-pyrrolidino]-8-(6,7-dimethoxy-1,2,3,4-tetrahydroisochinolin-2-yl)-4-morpholino-pyrimido[5,4-d]pyrimidin

Ausbeute: 77 % der Theorie,
Schmelzpunkt: 173-174 °C

| $C_{27}H_{36}N_8O_3$ (520,69) | | | | | | |
|---|---|---|---|---|---|---|
| Ber.:<br>Gef.: | C | 62,29<br>62,08 | H | 6,97<br>7,15 | N | 21,52<br>21,30 |

Beispiel 2

2-[4-(N,N-diethylamino)-piperidino)-8-(N-benzyl-N-methyl-amino)-4-morpholino-pyrimido[5,4-d]pyrimidin

1,73 g 2-(4-Oxo-piperidin-1-yl)-8-(N-benzyl-N-methyl-amino)-4-morpholino-pyrimido[5,4-d]pyrimidin werden in 20 ml Methanol und 20 ml Dioxan gelöst und mit 0,293 g Diethylamin versetzt. Zu dieser Lösung gibt man 0,31 g Natriumcyanborhydrid und 0,24 g Eisessig und rührt 7 Stunden bei Raumtemperatur. Dann wird die Reaktionsmischung in 100 ml Wasser eingerührt und mit 2N Natronlauge alkalisch gestellt. Diese Lösung wird mit Essigester extrahiert, die organische Phase wird abgetrennt, getrocknet und einrotiert. Das Produkt wird über eine Kieselgelsäule mit Essigester/Methanol/Ammoniak = 7:3:0,15 gereinigt.

Ausbeute: 570 mg (29 % der Theorie),
Öl;

| $C_{27}H_{38}N_8O$ (490,66) | | | | | | |
|---|---|---|---|---|---|---|
| Ber.:<br>Gef.: | C | 66,09<br>66,29 | H | 7,81<br>7,82 | N | 22,84<br>22,95 |

Analog werden hergestellt:

(1) 2-(4-Benzylamino-piperidino)-8-(N-benzyl-N-methyl-amino)-4-morpholino-pyrimido[5,4-d]pyrimidin

Ausbeute: 90 % der Theorie,
Schmelzpunkt: 103-106 °C

| $C_{30}H_{36}N_8O$ (524,67) | | | | | | |
|---|---|---|---|---|---|---|
| Ber.:<br>Gef.: | C | 68,68<br>68,39 | H | 6,92<br>7,02 | N | 21,36<br>21,24 |

(2) 8-(N-Benzyl-N-methyl-amino)-2-(4-ethylamino-piperidino)-4-morpholino-pyrimido[5,4-d]pyrimidin

Ausbeute: 65 % der Theorie,
Schmelzpunkt: 92-94 °C

| $C_{25}H_{34}N_8O$ (462,60) | | | | | | |
|---|---|---|---|---|---|---|
| Ber.:<br>Gef.: | C | 64,91<br>64,95 | H | 7,41<br>7,61 | N | 24,22<br>24,56 |

(3) 2-[4-(2,2-Diphenylethylamino)-piperidino]-4-(N-benzyl-N-methylamino)-8-morpholino-pyrimido[5,4-d]-pyrimidin

Ausbeute: 61 % der Theorie,
Schmelzpunkt: 106-108 °C

| $C_{37}H_{42}N_8O$ (614,80) | | | | | | |
|---|---|---|---|---|---|---|
| Ber.:<br>Gef.: | C | 72,29<br>71,92 | H | 6,89<br>6,98 | N | 18,23<br>18,00 |

(4) 2-[4-(3,3-Diphenylpropylamino)-piperidino]-4-(N-benzyl-N-methylamino)-8-morpholino-pyrimido[5,4-d]-pyrimidin

Ausbeute: 52 % der Theorie,
Schmelzpunkt: 56-58 °C

| $C_{38}H_{44}N_8O$ (628,83) | | | | | | |
|---|---|---|---|---|---|---|
| Ber.:<br>Gef.: | C | 72,58<br>72,53 | H | 7,05<br>7,13 | N | 17,82<br>18,21 |

(5) 2-[4-(N',N'-Dimethylcarbamoylmethyl-N-methylamino)-piperidino]-8-(N-benzyl-N-methyl-amino)-4-morpholino-pyrimido[5,4-d]pyrimidin

Aus 2-(4-Oxo-piperidin-1-yl)-8-(N-benzyl-N-methyl-amino)-4-morpholino-pyrimido[5,4-d]pyrimidin und N-Methylglycin-(N',N'-dimethyl)-amid.

Ausbeute: 60 % der Theorie,
Öl;

| $C_{28}H_{39}N_9O_2$ (533,68) | | | | | | |
|---|---|---|---|---|---|---|
| Ber.:<br>Gef.: | C | 63,02<br>62,94 | H | 7,37<br>7,50 | N | 23,62<br>23,72 |

(6) 2-(4-Amino-piperidino)-8-(N-benzyl-N-methyl-amino)-4-morpholino-pyrimido[5,4-d]pyrimidin-oxalat

Ausbeute: 24 % der Theorie,
Schmelzpunkt: 204 °C (Zers.)

| $C_{23}H_{30}N_8O$ x $(COOH)_2$ (524,58) | | | | | | |
|---|---|---|---|---|---|---|
| Ber.:<br>Gef.: | C | 57,24<br>57,17 | H | 6,15<br>6,31 | N | 21,36<br>21,53 |

(7) 2-[4-[N-[2-(N',N'-Dimethylamino)-ethyl]-N-methyl-amino]-piperidino]-8-(N-benzyl-N-methyl-amino)-4-morpholino-pyrimido[5,4-d]pyrimidin

Ausbeute: 60 % der Theorie,
Öl;

| $C_{28}H_{41}N_9O_2$ (519,70) | | | | | | |
|---|---|---|---|---|---|---|
| Ber.:<br>Gef.: | C | 64,71<br>64,84 | H | 7,95<br>8,05 | N | 24,26<br>24,42 |

(8) 2-[4-(N-methylamino)-piperidino]-8-(6,7-dimethoxy-1,2,3,4-tetrahydroisochinolin-2-yl)-4-morpholino-pyrimido[5,4-d]pyrimidin

Ausbeute: 31 % der Theorie,
Schmelzpunkt: 94 °C

| $C_{27}H_{36}N_8O_3$ (520,64) | | | | | | |
|---|---|---|---|---|---|---|
| Ber.:<br>Gef.: | C | 62,29<br>62,23 | H | 6,97<br>7,02 | N | 21,52<br>21,62 |

(9) 2-(4-Aminopiperidino)-8-(6,7-dimethoxy-1,2,3,4-tetrahydroisochinolin-2-yl)-4-morpholino-pyrimido[5,4-d]pyrimidin

Ausbeute: 22 % der Theorie,
Schmelzpunkt: 236-238 °C

| $C_{26}H_{34}N_8O_3$ (506,61) | | | | | | |
|---|---|---|---|---|---|---|
| Ber.:<br>Gef.: | C | 61,64<br>62,16 | H | 6,76<br>6,60 | N | 22,12<br>22,20 |

(10) 2-[4-(N,N-Diethylamino)-piperidino]-8-(6,7-dimethoxy-1,2,3,4-tetrahydroisochinolin-2-yl)-4-morpholino-pyrimido[5,4-d]pyrimidin

Ausbeute: 4 % der Theorie,
Schmelzpunkt: 122 °C

| $C_{30}H_{42}N_8O_3$ (562,72) | | | | | | |
|---|---|---|---|---|---|---|
| Ber.:<br>Gef.: | C | 64,03<br>63,91 | H | 7,62<br>7,38 | N | 19,91<br>20,02 |

(11) 2-[4-(Morpholin-4-yl)-piperidino]-8-(6,7-dimethoxy-1,2,3,4-tetrahydroisochinolin-2-yl)-4-morpholino-pyrimido[5,4-d]pyrimidin

Ausbeute: 58 % der Theorie,
Schmelzpunkt: 83 °C

| $C_{30}H_{40}N_8O_4$ (576,70) | | | | | | |
|---|---|---|---|---|---|---|
| Ber.:<br>Gef.: | C | 62,48<br>62,01 | H | 6,99<br>7,21 | N | 19,43<br>19,62 |

(12) 2-[4-(1-Oxidothiomorpholin-4-yl)-piperidino)-8-(6,7-dimethoxy-1,2,3,4-tetrahydroisochinolin-2-yl)-4-morpholino-pyrimido-[5,4-d]pyrimidin

Ausbeute: 40 % der Theorie,
Schmelzpunkt: 113 °C

| $C_{30}H_{40}N_8O_4S$ (608.77) | | | | | | |
|---|---|---|---|---|---|---|
| Ber.:<br>Gef.: | C | 59,14<br>59,10 | H | 6,62<br>6,62 | N | 18,41<br>18,59 |

Beispiel 3

8-(N-Benzyl-N-methyl-amino)-4-morpholino-2-(4-oxo-piperidin-1-yl)-pyrimido[5,4-d]pyrimidin

0,8 g 2-(8-Aza-1,4-dioxaspiro[4,5]decan-8-yl)-8-(N-benzyl-N-methyl-amino)-4-morpholino-pyrimido[5,4-d]pyrimidin werden in 40 ml 1N HCl 20 Minuten lang bei Raumtemperatur und anschließend 10 Minuten lang auf dem Dampfbad gerührt. Nach dem Abkühlen wird mit Natronlauge leicht basisch gestellt, mit Essigester extrahiert und die organische Phase nach Waschen mit Wasser und Trocknen über Natriumsulfat im Vakuum eingeengt. Der erhaltene Rückstand wird mit Ether verrieben.

Ausbeute: 0,6 g (82 % der Theorie),

| $C_{23}H_{27}N_7O_2$ (433,53) | | | | | | |
|---|---|---|---|---|---|---|
| Ber.:<br>Gef.: | C | 64,42<br>64,35 | H | 6,28<br>6,10 | N | 22,62<br>22,41 |

Analog wird hergestellt:

8-(6,7-Dimethoxy-1,2,3,4-tetrahydroisochinolin-2-yl)-2-(4-oxopiperidin-1-yl)-4-morpholino-pyrimido[5,4-d]-pyrimidin
Ausbeute: 76 % der Theorie,
$R_f$-Wert: 0,16 (Kieselgel; Essigsäureethylester/Cyclohexan = 2:1)

Beispiel 4

2-[4-(Carboxymethylamino)-piperidino]-8-(N-benzyl-N-methyl-amino)-4-morpholino-pyrimido[5,4-d]pyrimidin-Natriumsalz

0,5 g 2-[4-(Methyloxycarbonylmethylamino)-piperidino]-8-(N-benzyl-N-methyl-amino)-4-morpholino-pyrimido[5,4-d]pyri midin werden in 10 ml Methanol und 1 ml 1N NaOH ca. 15 Minuten lang auf dem Dampfbad gerührt. Anschließend wird im Vakuum eingeengt, mit Wasser verrieben und das erhaltene Produkt abgesaugt und getrocknet.
Ausbeute: 0,3 g (62 % der Theorie),
Schmelzpunkt: 166-169 ° C

| $C_{25}H_{32}N_8O_3Na$ (514,56) | | | | | | |
|---|---|---|---|---|---|---|
| Ber.:<br>Gef.: | C | 58,24<br>58,95 | H | 6,26<br>6,07 | N | 21,73<br>21,77 |

Analog wird hergestellt:

(1) 2-[4-(N,N-Dimethylamino)-piperidino)-8-(N-benzyl-N-carboxymethyl-amino)-4-morpholino-pyrimido[5,4-d]pyrimidin
Ausbeute: 79 % der Theorie,
Schmelzpunkt: 151-155 ° C

| $C_{26}H_{34}N_8O_3$ (506,61) | | | | | | |
|---|---|---|---|---|---|---|
| Ber.:<br>Gef.: | C | 61,64<br>61,44 | H | 6,76<br>7,10 | N | 22,12<br>22,37 |

Beispiel 5

2-[4-(N,N-Dimethylamino)-piperidino]-8-[N-benzyl-N-(3-benzoylaminopropyl)-amino]-4-morpholino-pyrimido-[5,4-d]pyrimidin

0,75 g 2-[4-(N,N-Dimethylamino)-piperidino)-8-[N-benzyl-N-(3-aminopropyl)-amino]-4-morpholino-pyrimido[5,4-d]pyrimidin werden in 8 ml Methylenchlorid gelöst und mit 0,24 g Benzoylchlorid und 0,3 g Triethylamin in 2 ml Methylenchlorid versetzt. Die Reaktionsmischung wird 12 Stunden bei Raumtemperatur gerührt und dann dreimal mit Wasser extrahiert. Die organische Phase wird über Natriumsulfat getrocknet und einrotiert. Der Rückstand wird mit Methylenchlorid/Methanol = 7:3 über eine Kieselgelsäule chromatographiert.
Ausbeute: 0,7 g (77 % der Theorie),
Öl;

| $C_{34}H_{43}N_9O_2$ (609,78) | | | | | | |
|---|---|---|---|---|---|---|
| Ber.:<br>Gef.: | C | 66,97<br>66,85 | H | 7,11<br>6,98 | N | 20,67<br>20,71 |

Analog werden folgende Verbindungen erhalten:

(1) 8-[N-Benzyl-N-(3-acetylaminopropyl)-amino]-2-[4-(N,N-dimethylamino)-piperidino]-4-morpholino-pyrimido[5,4-d]pyrimidin

Ausbeute: 69 % der Theorie,
Schmelzpunkt: 165-167 °C

| $C_{29}H_{41}N_9O_2$ (547,71) | | | | | | |
|---|---|---|---|---|---|---|
| Ber.:<br>Gef.: | C | 63,60<br>63,33 | H | 7,54<br>7,68 | N | 23,02<br>23,20 |

(2) 2-[4-(N,N-Dimethylamino)-piperidino]-8-[N-benzyl-N-(3-toluolsulfonamidopropyl)-amino]-4-morpholino-pyrimido[5,4-d]pyrimidin

Ausbeute: 77 % der Theorie,
Schmelzpunkt: 86-89 °C

| $C_{34}H_{45}N_9O_3S$ (659,86) | | | | | | | | |
|---|---|---|---|---|---|---|---|---|
| Ber.:<br>Gef.: | C | 61,89<br>61,79 | H | 6,87<br>6,91 | N | 19,10<br>18,92 | S | 4,87<br>5,28 |

Beispiel 6

8-[N-Benzyl-N-(3-aminopropyl)-amino]-2-[4-(N,N-dimethylamino)piperidino]-4-morpholino-pyrimido[5,4-d]-pyrimidin

4 g 8-[N-Benzyl-N-(2-cyano-ethyl)-amino]-2-[4-(N,N-dimethylamino)-piperidino]-4-morpholino-pyrimido-[5,4-d]-pyrimidin werden in 200 ml ethanolischem Ammoniak und in Gegenwart von 0,8 g Raney-Nickel mit Wasserstoff behandelt. Nach Aufnahme der berechneten Wasserstoffmenge wird vom Katalysator abfiltriert, das Filtrat im Vakuum eingeengt und der erhaltene Rückstand über eine Kieselgelsäule mit Methylenchlorid/Methanol/Ammoniak = 7:3:0,15 gereinigt.

Ausbeute: 2 g (50 % der Theorie),
Schmelzpunkt: 102-105 °C

| $C_{27}H_{39}N_9O$ (505,67) | | | | | | |
|---|---|---|---|---|---|---|
| Ber.:<br>Gef.: | C | 64,13<br>64,39 | H | 7,77<br>8,00 | N | 24,93<br>25,04 |

Beispiel I

Dragées mit 75 mg 2-[4-(N,N-Dimethylamino)-piperidino]-8-(6,7-dimethoxy-1,2,3,4-tetrahydro-isochinolin-2-yl)-4-morpholino-pyrimido[5,4-d]pyrimidin

| 1 Dragéekern enthält: | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Dragéekerne werden mit einem Film überzogen, der im wesentlichen aus Hydroxypropylmethylcellulose besteht. Die fertigen Filmdragées werden mit Bienenwachs geglänzt.

| | |
|---|---|
| Dragéegewicht: | 245 mg. |

Beispiel II

Tabletten mit 100 mg 2-[4-(N,N-Dimethylamino)-piperidino]-8-(6,7-dimethoxy-1,2,3,4-tetrahydro-isochinolin-2-yl)-4-morpholino-pyrimido[5,4-d]pyrimidin

Zusammensetzung:

| 1 Tablette enthält: | |
|---|---|
| Wirkstoff | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50 °C wird erneut gesiebt (1,5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

Beispiel III

Tabletten mit 150 mg 2-[4-(N,N-Dimethylamino)-piperidino]-8-(6,7-dimethoxy-1,2,3,4-tetrahydro-isochinolin-2-yl)-4-morpholino-pyrimido[5,4-d]pyrimidin

Zusammensetzung:

| 1 Tablette enthält: | |
|---|---|
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1,0 mg |
| | 300,0 mg |

Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen. Das bei 45 °C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

Beispiel IV

Hartgelatine-Kapseln mit 150 mg 2-[4-(N,N-Dimethylamino)-piperidino]-8-(6,7-dimethoxy-1,2,3,4-tetrahydro-isochinolin-2-yl)-4-morpholino-pyrimido[5,4-d]pyrimidin

| 1 Kapsel enthält: | |
|---|---|
| Wirkstoff | 150,0 mg |
| Maisstärke getr. | ca. 180,0 mg |
| Milchzucker pulv. | ca. 87,0 mg |
| Magnesiumstearat | 3,0 mg |
| | ca. 420,0 mg |

Herstellung:

Der Wirkstoff wird mit den Hilfsstoffen vermengt, durch ein Sieb von 0,75 mm-Maschenweite gegeben und in einem geeigneten Gerät homogen gemischt.
Die Endmischung wird in Hartgelatine-Kapseln der Größe 1 abgefüllt.

| | |
|---|---|
| Kapselfüllung: | ca. 320 mg |
| Kapselhülle: | Hartgelatine-Kapsel Größe 1. |

Beispiel V

Suppositorien mit 150 mg 2-[4-(N,N-Dimethylamino)-piperidino]-8-(6,7-dimethoxy-1,2,3,4-tetrahydro-isochinolin-2-yl)-4-morpholino-pyrimido[5,4-d]pyrimidin

| 1 Zäpfchen enthält: | |
|---|---|
| Wirkstoff | 150,0 mg |
| Polyäthylenglykol 1500 | 550,0 mg |
| Polyäthylenglykol 6000 | 460,0 mg |
| Polyoxyäthylensorbitanmonostearat | 840,0 mg |
| | 2 000,0 mg |

Herstellung:

Nach dem Aufschmelzen der Suppositorienmasse wird der Wirkstoff darin homogen verteilt und die Schmelze in vorgekühlte Formen gegossen.

Beispiel VI

Suspension mit 50 mg 2-[4-(N,N-Dimethylamino)-piperidino]-8-(6,7-dimethoxy-1,2,3,4-tetrahydro-isochinolin-2-yl)-4-morpholino-pyrimido[5,4-d]pyrimidin

| 100 ml Suspension enthalten: | |
|---|---|
| Wirkstoff | 1,0 g |
| Carboxymethylcellulose-Na-Salz | 0,1 g |
| p-Hydroxybenzoesäuremethylester | 0,05 g |
| p-Hydroxybenzoesäurepropylester | 0,01 g |
| Rohrzucker | 10,0 g |
| Glycerin | 5,0 g |
| Sorbitlösung 70%ig | 20,0 g |
| Aroma | 0,3 g |
| Wasser dest. ad | 100 ml |

Herstellung:

Dest. Wasser wird auf 70 °C erhitzt. Hierin wird unter Rühren p-Hydroxybenzoesäuremethylester und -propylester wobei Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 50 mg Wirkstoff.

Beispiel VII

Ampullen mit 10 mg 2-[4-(N,N-Dimethylamino)-piperidino]-8-(6,7-dimethoxy-1,2,3,4-tetrahydro-isochinolin-2-yl)-4-morpholino-pyrimido[5,4-d]pyrimidin

Zusammensetzung:

| | |
|---|---|
| Wirkstoff<br>0,01 n Salzsäure s.q.<br>Aqua bidest ad | 10,0 mg<br><br>2,0 ml |

Herstellung:

Die Wirksubstanz wird in der erforderlichen Menge 0,01 n HCl gelöst, mit Kochsalz isotonisch gestellt, sterilfiltriert und in 2 ml Ampullen abgefüllt. Die Sterilisation erfolgt durch 20 minütiges Erhitzen auf 121 ° C.

Beispiel VIII

Ampullen mit 50 mg 2-[4-(N,N-Dimethylamino)-piperidino]-8-(6,7-dimethoxy-1,2,3,4-tetrahydro-isochinolin-2-yl)-4-morpholino-pyrimido[5,4-d]pyrimidin

Zusammensetzung:

| | |
|---|---|
| Wirkstoff<br>0,01 n Salzsäure s.q.<br>Aqua bidest ad | 50,0 mg<br><br>10,0 ml |

Herstellung:

Die Wirksubstanz wird in der erforderlichen Menge 0,01 n HCl gelöst, mit Kochsalz isotonisch gestellt, sterilfiltriert und in 10 ml Ampullen abgefüllt. Die Sterilisation erfolgt durch 20 minütiges Erhitzen auf 121 ° C.

Beispiel IX

Trockenampullen mit 10 mg Doxorubicin und 10 mg 2-[4-(N,N-Dimethylamino)-piperidino]-8-(6,7-dimethoxy-1,2,3,4-tetrahydroisochinolin-2-yl)-4-morpholino-pyrimido[5,4-d]pyrimidin

Zusammensetzung der Trockenampulle:

| | |
|---|---|
| Doxorubicin<br>Wirksubstanz | 10,0 mg<br>10,0 mg |

Herstellung:

Die beiden Wirksubstanzen werden in der erforderlichen Menge 0,01 n HCl gelöst, sterilfiltriert und lyophylisiert.

Die Lösungsmittelampulle enthält 5 ml Kochsalzlösung.

Vor der Anwendung wird das Lyophylisat in der sterilen physiologischen Kochsalzlösung gelöst.

Beispiel X

Trockenampullen mit 50 mg Doxorubicin und 50 mg 2-[4-(N,N-Dimethylamino)-piperidino]-8-(6,7-dimethoxy-1,2,3,4-tetrahydroisochinolin-2-yl)-4-morpholino-pyrimido[5,4-d]pyrimidin

Zusammensetzung der Trockenampulle:

| | |
|---|---|
| Doxorubicin | 50,0 mg |
| Wirksubstanz | 50,0 mg |

Herstellung:

Die beiden Wirksubstanzen werden in der erforderlichen Menge 0,01 n HCl gelöst, sterilfiltriert und lyophylisiert.

Die Lösungsmittelampulle enthält 25 ml Kochsalzlösung.

Vor der Anwendung wird das Lyophylisat in der sterilen physiologischen Kochsalzlösung gelöst.

Selbstverständlich können alle übrigen Verbindungen der allgemeinen Formel I als Wirkstoffe in den vorstehenden galenischen Zubereitungen eingesetzt werden.

**Patentansprüche**

1. Trisubstituierte Pyrimido[5,4-d]pyrimidine der allgemeinen Formel

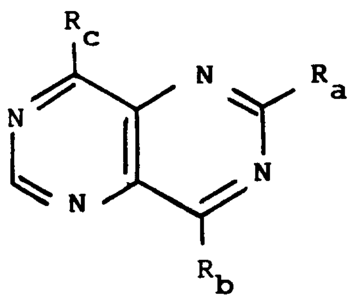

, (I)

in der
$R_a$ eine Pyrrolidino-, Piperidino- oder Hexamethyleniminogruppe, wobei eine Methylengruppe in einer Pyrrolidinogruppe in 3-Stellung oder in einer Piperidino- und Hexamethyleniminogruppe in 3- oder 4-Stellung jeweils durch eine $>CR_1$-A-($R_2NR_3$)-, >CO- oder >($R_4$O-C-O$R_5$)-Gruppe oder durch eine Gruppe der Formel

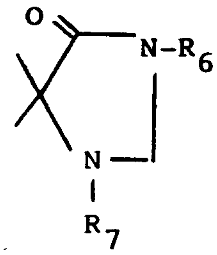

ersetzt ist, in denen
A eine Kohlenstoff-Stickstoff-Bindung oder eine Alkylengruppe mit 1 bis 3 Kohlenstoffatomen,
$R_1$ ein Wasserstoffatom oder eine Alkylgruppe mit 1 bis 3 Kohlenstoffatomen,
$R_2$ ein Wasserstoffatom oder eine gegebenenfalls durch eine Phenylgruppe substituierte Alkylgruppe

mit 1 bis 3 Kohlenstoffatomen,

$R_3$ ein Wasserstoffatom oder eine gegebenenfalls durch eine Phenylgruppe substituierte Alkylgruppe mit 1 bis 4 Kohlenstoffatomen, in der die Phenylgruppe durch ein Fluor-, Chlor- oder Bromatom, durch eine Alkyl- oder Alkoxygruppe mit jeweils 1 bis 3 Kohlenstoffatomen mono- oder disubstituiert sein kann, wobei die Substituenten gleich oder verschieden sein können,

eine durch eine Carboxy-, Alkoxycarbonyl-, Cyano-, Aminocarbonyl-, Alkylaminocarbonyl- oder Dialkylaminocarbonylgruppe oder durch zwei Phenylgruppen substituierte Alkylgruppe mit 1 bis 4 Kohlenstoffatomen, wobei die vorstehend erwähnten Alkyl- und Alkoxyteile jeweils 1 bis 3 Kohlenstoffatome enthalten können,

eine Alkylgruppe mit 2 bis 4 Kohlenstoffatomen, die in 2-, 3- oder 4-Stellung durch eine Hydroxy-, Amino-, Alkylamino- oder Dialkylaminogruppe substituiert sein kann, wobei die vorstehend erwähnten Alkylteile jeweils 1 bis 3 Kohlenstoffatome enthalten können,

eine Alkoxycarbonylgruppe mit insgesamt 2 bis 4 Kohlenstoffatomen oder

$R_2$ und $R_3$ zusammen mit dem dazwischenliegenden Stickstoffatom eine Cycloalkyleniminogruppe mit 3 bis 7 Ring gliedern, eine Morpholino-, Thiomorpholino-, 1-Oxido-thiomorpholino- oder 1,1-Dioxido-thiomorpholinogruppe,

$R_4$ und $R_5$, die gleich oder verschieden sein können, Alkylgruppen mit jeweils 1 bis 3 Kohlenstoffatomen oder

$R_4$ und $R_5$ zusammen eine n-Alkylengruppe mit 2 oder 3 Kohlenstoffatomen,

$R_6$ ein Wasserstoffatom oder eine Alkylgruppe mit 1 bis 3 Kohlenstoffatomen und

$R_7$ ein Wasserstoffatom, eine Alkylgruppe mit 1 bis 3 Kohlenstoffatomen oder eine Phenylgruppe darstellen,

$R_b$ und $R_c$, die gleich oder verschieden sein können, jeweils eine cyclische Alkylenimino- oder Alkenyleniminogruppe mit 5 bis 7 Ringgliedern, an die über die 2-, 3- oder 3-, 4-Stellungen eine 1,4-Butadienyl-brücke angefügt sein kann, welche durch eine Methylendioxygruppe monosubstituiert oder durch Fluor-, Chlor- oder Bromatome, durch Alkyl-, Alkoxy-, Amino-, Alkylamino-, Dialkylamino- oder Alkanoylaminogruppen mono-, di- oder trisubstituiert sein können, wobei die Alkyl- und Alkoxyteile jeweils 1 bis 3 Kohlenstoffatome und der Alkanoylteil 2 oder 3 Kohlenstoffatome enthalten können sowie die Substituenten gleich oder verschieden sein können, eine Morpholino-, Thiomorpholino-, 1-Oxido-thiomorpholino-, 1,1-Dioxidothiomorpholino- oder ($R_8NR_9$)-Gruppe, in der

$R_8$ ein Wasserstoffatom oder eine Alkylgruppe mit 1 bis 4 Kohlenstoffatomen, welche durch eine Phenyl-, Cyano-, Carboxy- oder Alkoxycarbonylgruppe oder in 2-, 3- oder 4-Stellung durch eine Amino-, Alkylamino-, Dialkylamino-, Alkanoylamino-, Benzoylamino- oder Phenylsulfonylaminogruppe substituiert sein kann, wobei die vorstehend erwähnten Alkyl-, Alkoxy- und Alkanoylteile jeweils 1 bis 3 Kohlenstoffatome enthalten können und der Phenylkern jeweils durch ein Fluor-, Chlor- oder Bromatom, durch eine Alkyl- oder Alkoxygruppe mit jeweils 1 bis 3 Kohlenstoffatome mono- oder disubstituiert sein und die Substituenten gleich oder verschieden sein können, und

$R_9$ ein Wasserstoffatom, eine gegebenenfalls durch eine Phenyl- oder Naphthylgruppe substituierte Alkylgruppe mit 1 bis 4 Kohlenstoffatomen, in der die Phenyl- und Naphthylgruppe jeweils durch ein Fluor-, Chlor- oder Bromatom, durch eine Alkyl-, Alkoxy-, Amino-, Alkylamino- oder Dialkylaminogruppe mono- oder disubstituiert sein kann, wobei die Substituenten gleich oder verschieden sein können und jeder Alkyl- und Alkoxyteil jeweils 1 bis 3 Kohlenstoffatomen enthalten kann, eine Alkanoylaminogruppe mit 1 bis 5 Kohlenstoffatomen oder eine Anthracenylgruppe darstellen,

oder $R_c$ auch eine gegebenenfalls durch eine Phenylgruppe substituierte Alkoxy- oder Alkylthiogruppe mit jeweils 1 bis 3 Kohlenstoffatomen bedeuten, und deren Salze.

**2.** Trisubstituierte Pyrimido[5,4-d]pyrimidine der allgemeinen Formel I gemäß Anspruch 1, in der
$R_a$ eine Pyrrolidino- oder Piperidinogruppe, in der eine Methylengruppe in 4-Stellung durch eine >$CR_1$-A-($R_2NR_3$)-, >CO- oder >($R_4$O-C-$OR_5$)-Gruppe oder durch eine Gruppe der Formel

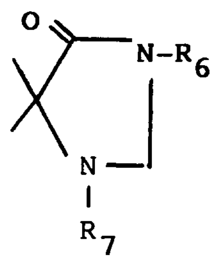

ersetzt ist, in denen
A eine Kohlenstoff-Stickstoff-Bindung oder eine Methylengruppe,
$R_1$ ein Wasserstoffatom,
$R_2$ ein Wasserstoffatom, eine Methyl- oder Ethylgruppe,
$R_3$ ein Wasserstoffatom,
eine gegebenenfalls durch eine Phenyl-, Carboxy-, Methoxycarbonyl-, Dimethylamino- oder Dimethylaminocarbonylgruppe oder durch zwei Phenylgruppen substituierte Alkylgruppe mit 1 bis 3 Kohlenstoffatomen,
eine Alkylgruppe mit 2 oder 3 Kohlenstoffatomen, die in 2- oder 3-Stellung durch eine Dimethylaminogruppe substituiert ist,
eine Alkoxycarbonylgruppe mit insgesamt 2 bis 4 Kohlenstoffatomen oder
$R_2$ und $R_3$ zusammen mit dem dazwischenliegenden Stickstoffatom eine Pyrrolidino- oder Piperidinogruppe,
$R_4$ und $R_5$ zusammen eine Ethylengruppe,
$R_6$ ein Wasserstoffatom und
$R_7$ eine Phenylgruppe darstellen,
$R_b$ eine Dialkylaminogruppe, in der jeder Alkylteil 1 bis 3 Kohlenstoffatome enthalten kann, eine Methylaminogruppe, die am Stickstoffatom durch eine Benzyl- oder Naphthylgruppe substituiert ist, eine Piperidino-, Morpholino- oder 1,2,3,4-Tetrahydro-isochinolyl-Gruppeund
$R_c$ eine gegebenenfalls am Stickstoffatom durch eine Alkylgruppe mit 1 bis 3 Kohlenstoffatomen substituierte Benzylaminogruppe, die im Phenylkern durch ein Fluor-, Chlor- oder Bromatom, durch eine Methyl- oder Methoxygruppe und im Alkylteil durch eine Phenyl-, Carboxy-, Methoxycarbonyl-, Ethoxycarbonyl- oder Cyanogruppe oder auch in 2- oder 3-Stellung durch eine Amino-, Acetylamino-, Benzoylamino- oder p-Toluolsulfonylaminogruppe substituiert sein kann, eine gegebenenfalls am Stickstoffatom durch eine Methylgruppe substituierte Naphthylaminogruppe, die im Naphthylkern durch eine Methoxy- oder Dimethylaminogruppe substituiert sein kann, eine gegebenenfalls durch ein Fluor-, Chlor- oder Bromatom, durch eine Methyl-, Methoxy-, Methylendioxy- oder Acetamidogruppe substituierte 1,2,3,4-Tetrahydro-isochinolyl-Gruppe, eine Morpholino-, N-Methyl-cyclohexyl-methylamino- oder Benzyloxygruppe bedeuten, und deren Salze.

**3.** Trisubstituierte Pyrimido[5,4-d]pyrimidine der allgemeinen Formel I gemäß Anspruch 1, in der
$R_a$ eine Piperidinogruppe, in der eine Methylengruppe in 4-Stellung durch eine $>CR_1$-A-($R_2NR_3$)-Gruppe ersetzt ist, in der
A eine Kohlenstoff-Stickstoff-Bindung oder eine Methylengruppe,
$R_1$ ein Wasserstoffatom,
$R_2$ ein Wasserstoffatom, eine Methyl- oder Ethylgruppe,
$R_3$ ein Wasserstoffatom,
eine durch zwei Phenylgruppen substituierte Alkylgruppe mit 2 oder 3 Kohlenstoffatomen,
eine Alkylgruppe mit 1 bis 3 Kohlenstoffatomen, die in 2- oder 3-Stellung durch eine Dimethylaminogruppe substituiert sein kann,
eine Methoxycarbonylgruppe oder
$R_2$ und $R_3$ zusammen mit dem dazwischenliegenden Stickstoffatom eine Piperidinogruppe darstellen,
$R_b$ eine Dimethylaminogruppe, in der eine Methylgruppe durch einen Benzyl- oder Naphthylmethylrest substituiert ist, eine Piperidino- oder Morpholinogruppe und
$R_c$ eine Dimethylaminogruppe, in der eine Methylgruppe durch einen gegebenenfalls im Phenylkern durch ein Fluor-, Chlor- oder Bromatom, durch eine Methyl- oder Methoxygruppe substituierten Benzylrest oder durch einen Naphthylmethylrest substituiert ist, eine 1,2,3,4-Tetrahydro-6,7-dimethoxy-isochinolinyl-, 1,2,3,4-Tetrahydro-6,7-methylendioxy-isochinolinyl-, Morpholino-oder N-(3-Benzoylamino-propyl)-benzylaminogruppe, wobei die Reste $R_b$ und $R_c$ verschieden sind, bedeuten, und deren Salze.

**4.** Folgende trisubstituierte Pyrimido[5,4-d]pyrimidine der allgemeinen Formel I gemäß Anspruch 1:
(a) 2-[4-(N,N-Dimethylamino)-piperidino]-8-(N-benzyl-N-methyl-amino)-4-morpholino-pyrimido[5,4-d]-pyrimidin,
(b) 2-[4-(N-Methoxycarbonyl-amino)-piperidino]-8-(N-benzyl-N-methyl-amino)-4-morpholino-pyrimido-[5,4-d]pyrimidin,
(c) 2-[4-(N,N-Dimethylamino)-piperidino]-8-(N-benzyl-N-methyl-amino)-4-piperidino-pyrimido[5,4-d]-pyrimidin,
(d) 8-(N-Benzyl-N-methyl-amino)-2-[4-(piperidino)-piperidin-1-yl]-4-morpholino-pyrimido[5,4-d]-pyrimidin,
(e) 2-[4-(N,N-Dimethylaminomethyl)-piperidino]-8-[N-(naphth-1-yl-methyl)-N-methyl-amino]-4-morpholino-pyrimido[5,4-d]pyrimidin,
(f) 4-(N-Benzyl-N-methyl-amino)-2-[4-(N,N-dimethylamino)-piperidino]-8-morpholino-pyrimido[5,4-d]-pyrimidin,
(g) 2-[4-(N,N-Dimethylamino)-piperidino]-8-(6,7-dimethoxy-1,2,3,4-tetrahydro-isochinolin-2-yl)-4-morpholino-pyrimido[5,4-d]pyrimidin,
(h) 2-[4-(N,N-Dimethylamino)-piperidino]-8-[N-(naphth-1-yl-methyl)-N-methyl-amino]-4-morpholino-pyrimido[5,4-d]pyrimidin,
(i) 2-[4-(2,2-Diphenylethylamino)-piperidino]-4-(N-benzyl-N-methyl-amino)-8-morpholino-pyrimido[5,4-d]pyrimidin,
(j) 2-[4-(N,N-Dimethylamino)-piperidino]-8-[N-benzyl-N-(3-benzoylaminopropyl)-amino]-4-morpholino-pyrimido[5,4-d]pyrimidin,
(k) 2-[4-(N,N-Dimethylamino)-piperidino]-4-[N-(naphth-1-yl-methyl)-N-methyl-amino]-8-morpholino-pyrimido[5,4-d]pyrimidin und deren Salze.

**5.** 2-[4-(N,N-Dimethylamino)-piperidino]-8-(6,7-dimethoxy-1,2,3,4-tetrahydro-isochinolin-2-yl)-4-morpholino-pyrimido[5,4-d]pyrimidin und dessen Salze.

**6.** Physiologisch verträgliche Salze der Verbindungen gemäß den Ansprüchen 1 bis 5.

**7.** Arzneimittel enthaltend eine Verbindung gemäß den Ansprüchen 1 bis 5 oder ein physiologisch verträgliches Salz gemäß Anspruch 6 neben einem oder mehreren inerten Trägerstoffen.

**8.** Arzneimittel gemäß Anspruch 7, dadurch gekennzeichnet, daß dieses zusätzlich noch ein Chemotherapeuticum enthält.

**9.** Verfahren zur Herstellung eines Arzneimittels gemäß Anspruch 7 oder 8, dadurch gekennzeichnet, daß eine Verbindung gemäß den Amsprüchen 1 bis 5 oder ein physiologisch verträgliches Salz gemäß Anspruch 6 gegebenenfalls in Kombination mit einem Chemotherapeuticum in einen oder mehrere inerte übliche Träger eingearbeitet wird.

**10.** Verwendung einer Verbindung gemäß den Ansprüchen 1 bis 6 zur Herstellung eines Arzneimittels mit einer sensibilisierenden Wirkung auf resistente Tumore.

**11.** Verfahren zur Herstellung der Verbindungen gemäß den Ansprüchen 1 bis 6, dadurch gekennzeichnet, daß
a) eine Verbindung der allgemeinen Formel

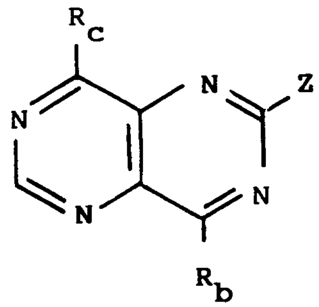

, (II)

in der
$R_b$ und $R_c$ wie in den Ansprüchen 1 bis 5 definiert sind und Z eine Austrittsgruppe darstellt, mit einer Verbindung der allgemeinen Formel

$R_a$ - H ,  (III)

in der
$R_a$ wie in den Ansprüchen 1 bis 5 definiert ist, umgesetzt wird oder
b) zur Herstellung von Verbindungen der allgemeinen Formel I, in der $R_a$ eine >CH-($R_2NR_3$)-Gruppe darstellt, eine Verbindung der allgemeinen Formel

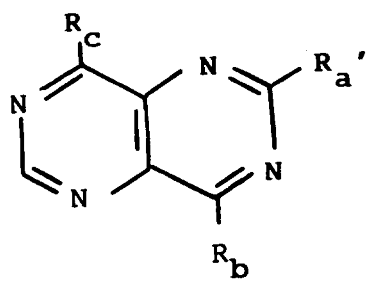

, (IV)

in der
$R_b$ und $R_c$ wie in den Ansprüchen 1 bis 5 definiert sind und $R_{a'}$ eine Pyrrolidino-, Piperidino- oder Hexamethyleniminogruppe darstellt, wobei die Methylengruppe der Pyrrolidinogruppe in 3-Stellung oder der Piperidino- oder Hexamethyleniminogruppe in 3- oder 4-Stellung durch eine >CO-Gruppe ersetzt ist, mit einem Amin der allgemeinen Formel

H - $R_2NR_3$ ,  (V)

in der
$R_2$ und $R_3$ wie in den Ansprüchen 1 bis 5 definiert sind, reduktiv aminiert wird und
gewünschtenfalls eine so erhaltene Verbindung der allgemeinen Formel I, die eine >($R_4OCOR_5$)-Gruppe enthält, mittels Hydrolyse in eine entsprechende Carbonylverbindung der allgemeinen Formel I übergeführt wird oder
eine so erhaltene Verbindung der allgemeinen Formel I, die eine Alkoxycarbonylgruppe enthält, mittels Hydrolyse in eine entsprechende Carboxyverbindung der allgemeinen Formel I übergeführt wird oder
eine so erhaltene Verbindung der allgemeinen Formel I, die eine Carboxygruppe enthält, mittels Veresterung oder Amidierung in eine entsprechende Verbindung der allgemeinen Formel I, die eine Alkoxycarbonyl-, Aminocarbonyl-, Alkylaminocarbonyl- oder Dialkylaminocarbonylgruppe enthält, übergeführt wird oder
eine so erhaltene Verbindung der allgemeinen Formel I, die eine Thiomorpholinogruppe enthält, mittels Oxidation in eine entsprechende 1-Oxido-thiomorpholinoverbindung der allgemeinen Formel I übergeführt wird oder
eine so erhaltene Verbindung der allgemeinen Formel I, die eine Thiomorpholino- oder 1-Oxido-thiomorpholinogruppe enthält, mittels Oxidation in eine entsprechende 1,1-Dioxido-thiomorpholinoverbindung der allgemeinen Formel I übergeführt wird oder

bei den vorstehend beschriebenen Umsetzungen gegebenenfalls zum Schutze von reaktiven Gruppen verwendete Schutzreste anschließend wieder abgespalten werden und

eine so erhaltene Verbindung in ihr Salz, insbesondere in ihr physiologisch verträgliches Salz, übergeführt wird.

Europäisches Patentamt

# EUROPÄISCHER RECHERCHENBERICHT

Nummer der Anmeldung
EP 94 11 1739

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | EP-A-0 023 559 (DR. KARL THOMAE GMBH) 11. Februar 1981<br>* Ansprüche 1-13 *<br>--- | 1-11 | C07D487/04<br>A61K31/505<br>//(C07D487/04, 239:00,239:00) |
| Y | EP-A-0 055 444 (DR. KARL THOMAE GMBH) 7. Juli 1982<br>* Ansprüche 1-7 *<br>--- | 1-11 | |
| Y | US-A-4 329 346 (BOEHRINGER INGELHEIM GMBH) 11. Mai 1982<br>* Ansprüche 1,2 *<br>--- | 1-11 | |
| Y | DE-A-34 23 092 (DR. KARL THOMAE) 2. Januar 1986<br>* Seite 6, Zeile 24 - Seite 6, Zeile 30; Ansprüche 1-14 *<br>----- | 1-11 | |

RECHERCHIERTE SACHGEBIETE (Int.Cl.6)

C07D
A61K

Der vorliegende Recherchenbericht wurde für alle Patentansprüche erstellt

| Recherchenort | Abschlußdatum der Recherche | Prüfer |
|---|---|---|
| MÜNCHEN | 22. Dezember 1994 | Herz, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)